# EUROPEAN PATENT SPECIFICATION

(11) **EP 2 744 773 B1**
(45) Date of publication and mention of the grant of the patent: **02.11.2016**
(21) Application number: 12762710.7
(22) Date of filing: 17.08.2012
(51) Int. Cl.: C07C 43/23, A61K 31/085, A61K 45/06, A61K 31/165, A61K 31/09, A61K 31/404, A61K 31/407, A61K 31/4166, A61K 31/497, A61K 39/395, A61K 31/337

(54) **FLUORINATED BISPHENOL ETHER COMPOUNDS AND METHODS FOR THEIR USE**
FLUORIERTE BISPHENOLETHERVERBINDUNGEN UND VERFAHREN ZU IHRER VERWENDUNG
COMPOSÉS D'ÉTHERS DE BISPHÉNOL FLUORÉS ET LEURS PROCÉDÉS D'UTILISATION

(30) Priority: 19.08.2011 US 201161525643 P
(43) Date of publication of application: 25.06.2014
(73) Proprietor: British Columbia Cancer Agency Branch, Vancouver, British Columbia V5Z 4E6 (CA); The University of British Columbia, Vancouver, BC V6T 1Z3 (CA)
(72) Inventor: ANDERSEN, Raymond, John, Vancouver, British Columbia V6T 1Z3 (CA); FERNANDEZ, Javier, Garcia, Vancouver, British Columbia V6T 1Z3 (CA); SADAR, Marianne, Dorothy, Vancouver, British Columbia V5Z 4E6 (CA); BANUELOS, Carmen, Adriana, Vancouver, British Columbia V5Z 4E6 (CA); MAWJI, Nasrin, Vancouver, British Columbia V5Z 4E6 (CA); WANG, Jun, Vancouver, British Columbia V5Z 4E6 (CA)
(74) Representative: Grund, Martin
(86) International application number: PCT/US2012/051481
(87) International publication number: WO 2013/028572

(56) References cited:
- WO-A1-2010/000066
- SATOH K ET AL: "Study on anti-androgenic effects of bisphenol a diglycidyl ether (BADGE), bisphenol F diglycidyl ether (BFDGE) and their derivatives using cells stably transfected with human androgen receptor, AR-EcoScreen", FOOD AND CHEMICAL TOXICOLOGY, PERGAMON, GB, vol. 42, no. 6, 1 June 2004 (2004-06-01), pages 983-993, XP002626868, ISSN: 0278-6915, DOI: 10.1016/J.FCT.2004.02.011 [retrieved on 2004-03-16]

## Description

### BACKGROUND

### Technical Field

This invention relates to therapeutics, their uses and compounds for use in methods for the treatment of various indications, including various cancers. In particular the invention relates to compounds for use in therapies and methods of treatment for cancers such as prostate cancer, including all stages and androgen dependent, androgen-sensitive and castration-resistant cancers (also referred to as hormone refractory, androgen-independent, androgen deprivation resistant, androgen ablation resistant, androgen depletion-independent, castration-recurrent, anti-androgen-recurrent).

### Description of the Related Art

Androgens mediate their effects through the androgen receptor (AR). Androgens play a role in a wide range of developmental and physiological responses and are involved in male sexual differentiation, maintenance of spermatogenesis, and male gonadotropin regulation (R. K. Ross, G. A. Coetzee, C. L. Pearce, J. K. Reichardt, P. Bretsky, L. N. Kolonel, B. E. Henderson, E. Lander, D. Altshuler & G. Daley, Eur Urol 35, 355-361 (1999); A. A. Thomson, Reproduction 121, 187-195 (2001); N. Tanji, K. Aoki & M. Yokoyama, Arch Androl 47, 1-7 (2001)). Several lines of evidence show that androgens are associated with the development of prostate carcinogenesis. Firstly, androgens induce prostatic carcinogenesis in rodent models (R. L. Noble, Cancer Res 37, 1929-1933 (1977); R. L. Noble, Oncology 34, 138-141 (1977)) and men receiving androgens in the form of anabolic steroids have a higher incidence of prostate cancer (J. T. Roberts & D. M. Essenhigh, Lancet 2, 742 (1986); J. A. Jackson, J. Waxman & A. M. Spiekerman, Arch Intern Med 149, 2365-2366 (1989); P. D. Guinan, W. Sadoughi, H. Alsheik, R. J. Ablin, D. Alrenga & I. M. Bush, Am J Surg 131, 599-600 (1976)). Secondly, prostate cancer does not develop if humans or dogs are castrated before puberty (J. D. Wilson & C. Roehrborn, J Clin Endocrinol Metab 84, 4324-4331 (1999); G. Wilding, Cancer Surv 14, 113-130 (1992)). Castration of adult males causes involution of the prostate and apoptosis of prostatic epithelium while eliciting no effect on other male external genitalia (E. M. Bruckheimer & N. Kyprianou, Cell Tissue Res 301, 153-162 (2000); J. T. Isaacs, Prostate 5, 545-557 (1984)). This dependency on androgens provides the underlying rationale for treating prostate cancer with chemical or surgical castration (androgen ablation).

Androgens also play a role in female cancers. One example is ovarian cancer where elevated levels of androgens are associated with an increased risk of developing ovarian cancer (K. J. Helzlsouer, A. J. Alberg, G. B. Gordon, C. Longcope, T. L. Bush, S. C. Hoffman & G. W. Comstock, JAMA 274, 1926-1930 (1995); R. J. Edmondson, J. M. Monaghan & B. R. Davies, Br J Cancer 86, 879-885 (2002)). The AR has been detected in a majority of ovarian cancers (H. A. Risch, JNatl Cancer Inst 90, 1774-1786 (1998); B. R. Rao & B. J. Slotman, Endocr Rev 12, 14-26 (1991); G. M. Clinton & W. Hua, Crit Rev Oncol Hematol 25, 1-9 (1997)), whereas estrogen receptor-alpha (ERa) and the progesterone receptor are detected in less than 50% of ovarian tumors.

The only effective treatment available for advanced prostate cancer is the withdrawal of androgens which are essential for the survival of prostate epithelial cells. Androgen ablation therapy causes a temporary reduction in tumor burden concomitant with a decrease in serum prostate-specific antigen (PSA). Unfortunately prostate cancer can eventually grow again in the absence of testicular androgens (castration-resistant disease) (Huber et al 1987 Scand J. Urol Nephrol. 104, 33-39). Castration-resistant prostate cancer is biochemically characterized before the onset of symptoms by a rising titer of serum PSA (Miller et al 1992 J. Urol. 147, 956-961). Once the disease becomes castration-resistant most patients succumb to their disease within two years.

The AR has distinct functional domains that include the carboxy-terminal ligand-binding domain (LBD), a DNA-binding domain (DBD) comprising two zinc finger motifs, and an N-terminus domain (NTD) that contains one or more transcriptional activation domains. Binding of androgen (ligand) to the LBD of the AR results in its activation such that the receptor can effectively bind to its specific DNA consensus site, termed the androgen response element (ARE), on the promoter and enhancer regions of "normally" androgen regulated genes, such as PSA, to initiate transcription. The AR can be activated in the absence of androgen by stimulation of the cAMP-dependent protein kinase (PKA) pathway, with interleukin-6 (IL-6) and by various growth factors (Culig et al 1994 Cancer Res. 54, 5474-5478; Nazareth et al 1996 J. Biol. Chem. 271, 19900-19907; Sadar 1999 J. Biol. Chem. 274, 7777-7783; Ueda et al 2002 A J. Biol. Chem. 277, 7076-7085; and Ueda et al 2002 B J. Biol. Chem. 277, 38087-38094). The mechanism of ligand-independent transformation of the AR has been shown to involve: 1) increased nuclear AR protein suggesting nuclear translocation; 2) increased AR/ARE complex formation; and 3) the AR-NTD (Sadar 1999 J. Biol. Chem. 274, 7777-7783; Ueda et al 2002 A J. Biol. Chem. 277, 7076-7085; and Ueda et al 2002 B J. Biol. Chem. 277, 38087-38094). The AR may be activated in the absence of testicular androgens by alternative signal transduction pathways in castration-resistant disease, which is consistent with the finding that nuclear AR protein is present in secondary prostate cancer tumors (Kim et al 2002 Am. J. Pathol. 160, 219-226; and van der Kwast et al 1991 Inter. J, Cancer 48, 189-193).

Available inhibitors of the AR include nonsteroidal antiandrogens such as bicalutamide (Casodex™), nilutamide, flutamide, investigational drugs MDV3100 and ARN-509, and the steroidal antiandrogen, cyproterone acetate. These antiandrogens target the LBD of the AR and predominantly fail presumably due to poor affinity and mutations that lead to activation of the AR by these same antiandrogens (Taplin, M.E., Bubley, G.J., Kom Y.J., Small E.J., Uptonm M., Rajeshkumarm B., Balkm S.P., CancerRes., 59, 2511-2515 (1999)). These antiandrogens would also have no effect on the recently discovered AR splice variants that lack the ligand-binding domain (LBD) to result in a constitutively active receptor which promotes progression of androgen-independent prostate cancer (Dehm SM, Schmidt LJ, Heemers HV, Vessella RL, Tindall DJ., Cancer Res 68, 5469-77, 2008; Guo Z, Yang X, Sun F, Jiang R, Linn DE, Chen H, Chen H, Kong X, Melamed J, Tepper CG, Kung HJ, Brodie AM, Edwards J, Qiu Y., Cancer Res. 69, 2305-13, 2009; Hu et al 2009 Cancer Res. 69, 16-22; Sun et al 2010 J Clin Invest. 2010 120, 2715-30).

Conventional therapy has concentrated on androgen-dependent activation of the AR through its C-terminal domain. Recent studies developing antagonists to the AR have concentrated on the C-terminus and specifically: 1) the allosteric pocket and AF-2 activity (Estebanez-Perpina et al 2007, PNAS 104, 16074-16079); *2) in silico* "drug repurposing" procedure for identification of nonsleroidal antagonists (Bisson et al 2007, PNAS 104, 11927 - 11932); and coactivator or corepressor interactions (Chang et al 2005, Mol Endocrinology 19, 2478-2490; Hur et al 2004, PLoS Biol 2, E274; Estébanez-Perpiña et al 2005, JBC 280, 8060-8068; He et al 2004, Mol Cell 16, 425-438).

The AR-NTD is also a target for drug development (*e*.*g*., WO 2000/001813), since the NTD contains Activation-Function-1 (AF-1) which is the essential region required for AR transcriptional activity (Jenster et al 1991. Mol Endocrinol. 5, 1396-404). The AR-NTD importantly plays a role in activation of the AR in the absence of androgens (Sadar, M.D. 1999 J. Biol. Chem. 274, 7777-7783; Sadar MD et al 1999 Endocr Relat Cancer. 6, 487-502; Ueda et al 2002 J. Biol. Chem. 277, 7076-7085; Ueda 2002 J. Biol. Chem. 277, 38087-38094; Blaszczyk et al 2004 Clin Cancer Res. 10, 1860-9; Dehm et al 2006 J Biol Chem. 28, 27882-93; Gregory et al 2004 J Biol Chem. 279, 7119-30). The AR-NTD is important in hormonal progression of prostate cancer as shown by application of decoy molecules (Quayle et al 2007, Proc Natl Acad Sci USA. 104,1331-1336).

While the crystal structure has been resolved for the AR C-terminus LBD, this has not been the case for the NTD due to its high flexibility and intrinsic disorder in solution (Reid et al 2002 J. Biol. Chem. 277, 20079-20086) thereby hampering virtual docking drug discovery approaches.

Recent advances in the development of compounds that modulate AR include the bis-phenol compounds disclosed in published PCT WO 2010/000066 to the British Columbia Cancer Agency Branch and The University of British Columbia. While such compounds appear promising, there remains a need in the art for additional and/or improved compounds that modulate the AR, and which provide treatment for conditions that benefit from such modulation.

### BRIEF SUMMARY

The invention is defined by the appended claims.

The compounds described herein may be used for *in vivo* or *in vitro* research uses (*i.e.,* non-clinical) to investigate the mechanisms of orphan and nuclear receptors (including steroid receptors such as the androgen receptor). Furthermore, these compounds may be used individually or as part of a kit for *in vivo* or *in vitro* research to investigate signal transduction pathways and/or the activation of orphan and nuclear receptors using recombinant proteins, cells maintained in culture, and/or animal models.

This invention is also based in part on the surprising discovery that the compounds described herein, may also be used to modulate AR activity either *in vivo* or *in vitro* for both research and therapeutic uses. In particular, experiments conducted in support of the present invention show that the presence of a chlorine moiety and at least one fluorine moiety in the disclosed compounds imparts unexpectedly improved activity compared to other known AR modulators (see e.g., Examples 5-9). The compounds may be used in an effective amount so that androgen receptor activity may be modulated. The AR may be mammalian, for example human. In particular, the compounds may be used to inhibit the AR. The compounds modulatory activity may be used in either an *in vivo* or an *in vitro* model for the study of at least one of the following indications: prostate cancer, breast cancer, ovarian cancer, salivary gland carcinoma, endometrial cancer, hair loss, acne, hirsutism, ovarian cysts, polycystic ovary disease, precocious puberty (testoxicosis), spinal and bulbar muscular atrophy (SBMA, Kennedy's disease), and age-related macular degeneration. Furthermore, the compounds modulatory activity may be used for the treatment of at least one of the following indications: prostate cancer, breast cancer, ovarian cancer, endometrial cancer, salivary gland carcinoma, hair loss, acne, hirsutism, ovarian cysts, polycystic ovary disease, precocious puberty, spinal and bulbar muscular atrophy, and age-related macular degeneration. The indication for treatment may be prostate cancer. The prostate cancer may be castration-resistant prostate cancer. The prostate cancer may be androgen-dependent prostate cancer. In other examples the indication is Kennedy's disease.

In accordance with one embodiment, there is provided a compound having a structure of Formula I: 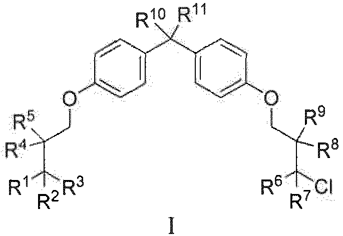 or a pharmaceutically acceptable salt or stereoisomer thereof, wherein R¹, R², R³, R⁴, R⁵, R⁶, R⁷, R⁸, R⁹, R¹⁰ and R¹¹ are as defined by the appended claims, and wherein at least one of R¹, R², R³, R⁴ or R⁵ is F.

In other embodiments, the present disclosure provides the use of a compound of Formula I, for modulating androgen receptor (AR) activity. Methods for modulating AR, as well as pharmaceutical compositions comprising a compound of Formula I and a pharmaceutically acceptable excipient are also provided.

In addition, the present disclosure provides combination therapy treatments for any of the disease states disclosed herein, for example prostate cancer. The disclosed therapies include use of a pharmaceutical composition comprising a compound of Formula I, an additional therapeutic agent and a pharmaceutically acceptable excipient. Methods and compositions related to the same are also provided.

These and other aspects of the invention will be apparent upon reference to the following detailed description. To this end, various references are set forth herein which describe in more detail certain background information, procedures, compounds and/or compositions.

### BRIEF DESCRIPTION OF THE DRAWINGS

Figures not pertaining to the invention are for illustrative purposes only.

In the figures, identical reference numbers identify similar elements. The sizes and relative positions of elements in the figures are not necessarily drawn to scale and some of these elements are arbitrarily enlarged and positioned to improve figure legibility. Further, the particular shapes of the elements as drawn are not intended to convey any information regarding the actual shape of the particular elements, and have been solely selected for ease of recognition in the figures.
Figures 1A and 1B depict inhibition of androgen dependent proliferation by a representative compound of the invention and a comparative compound.
Figure 2 shows inhibition of androgen receptor N-terminal domain transactivation by a representative compound of the invention and a comparative compound.
Figure 3 presents data showing tumor regression.
Figure 4 depicts change in tumor volume for each mouse.
Figure 5 is a plot showing change in body weight after treatment with representative compounds of the invention and a comparative compound.
Figure 6 shows tumor volume as function of time after first injection with comparative compounds.
Figure 7 presents tumor volume data.
Figure 8 shows change in tumor volume for each mouse treated with comparative compounds.
Figure 9 presents change in body weight data for mice treated with comparative compounds.

### DETAILED DESCRIPTION

The invention is defined by the appended claims.

### I. Definitions

In the following description, certain specific details are set forth in order to provide a thorough understanding of various embodiments. However, one skilled in the art will understand that the invention may be practiced without these details. In other instances, well-known structures have not been shown or described in detail to avoid unnecessarily obscuring descriptions of the embodiments. Unless the context requires otherwise, throughout the specification and claims which follow, the word "comprise" and variations thereof, such as, "comprises" and "comprising" are to be construed in an open, inclusive sense, that is, as "including, but not limited to." Further, headings provided herein are for convenience only and do not interpret the scope or meaning of the claimed invention.

Reference throughout this specification to "one embodiment" or "an embodiment" means that a particular feature, structure or characteristic described in connection with the embodiment is included in at least one embodiment. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments. Also, as used in this specification and the appended claims, the singular forms "a," "an," and "the" include plural referents unless the content clearly dictates otherwise. It should also be noted that the term "or" is generally employed in its sense including "and/or" unless the content clearly dictates otherwise.

The terms below, as used herein, have the following meanings, unless indicated otherwise:
"Alkyl" refers to a straight or branched hydrocarbon chain radical which is saturated having from one to twelve carbon atoms, and which is attached to the rest of the molecule by a single bond. Alkyls comprising any number of carbon atoms from 1 to 12 are included. An alkyl comprising up to 5 carbon atoms is a C₁-C₅ alkyl. A C₁-C₅ alkyl includes C₅ alkyls, C₄ alkyls, C₃ alkyls, C₂ alkyls and C₁ alkyl (*i.e*., methyl).

Non-limiting examples of saturated C₁-C₅ alkyl include methyl, ethyl, n-propyl, i-propyl, sec-propyl, n-butyl, i-butyl, sec-butyl, t-butyl and n-pentyl. Non-limiting examples of C₂-C₅ alkenyl include vinyl, allyl, isopropenyl, 1-propene-2-yl, 1-butene-l-yl, 1-butene-2-yl, 1-butene-3-yl, 2-butene-1-yl, 2-butene-2-yl, penteneyl and the like. Non-limiting examples of C₂-C₅ alkynyl include ethynyl, propynyl, butynyl, pentynyland the like.

"Hydroxy" or "hydroxyl" refers to the -OH radical.

"Fluoro" and "chloro" refer to fluorine (F) and chlorine (Cl) substituents, respectively, and also include radioisotopes of the same.

As used herein, the symbol " 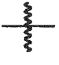 " (hereinafter may be referred to as "a point of attachment bond") denotes a bond that is a point of attachment between two chemical entities, one of which is depicted as being attached to the point of attachment bond and the other of which is not depicted as being attached to the point of attachment bond. For example, " 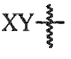 " is not depicted as being attached to the point of attachment bond. For example, "^{XY}" indicates that the chemical entity "XY" is bonded to another chemical entity via the point of attachment bond. Furthermore, the specific point of attachment to the non-depicted chemical entity may be specified by inference. For example, the compound CH₃-R³, wherein R³ is H or " 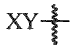 " infers that when R³ is "XY", the point of attachment bond is the same bond as the bond "infers that when R³ is "XY", the point of attachment bond is the same bond as the bond by which R³ is depicted as being bonded to CH₃.

### II. Compounds and Methods

As noted above, the present disclosure provides a compound having a structure of Formula I: 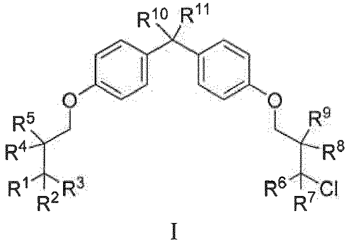 or a pharmaceutically acceptable salt or stereoisomer thereof, wherein:
R¹ is F, OH or OY;
R², R³ R⁵, R⁶, R⁷ and R⁹ are each independently H or F;
R⁴ is H, F, OH or OY;
R⁸ is OH or OY;
R¹⁰ and R¹¹ are each independently F or C₁-C₅ alkyl; and
Y is a moiety from Table I;
wherein at least one of R¹, R², R³, R⁴ or R⁵ is F

In another embodiment, the compound has one of the following structures (Ia), (Ib), (Ic) or (Id):

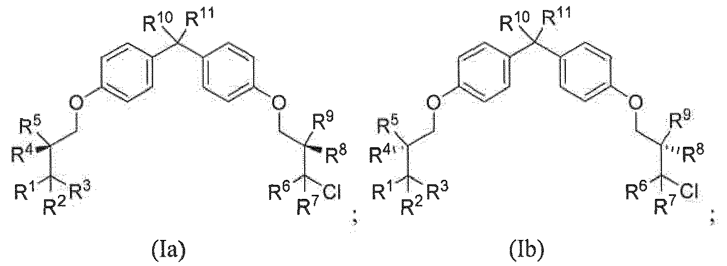

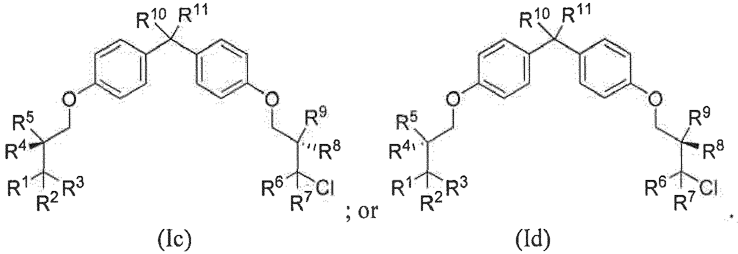

In some embodiments of any of the foregoing embodiments, R¹ is F, and in other embodiments R¹ is OH. In yet other embodiments, at least one of R¹, R⁴ or R⁸ is OY.

In some other embodiments of any of the foregoing embodiments, each of R⁶ and R⁷ is H.

In some other embodiments of any of the foregoing embodiments, R⁸ is OH, and in some other embodiments R⁹ is H. In certain embodiments, R⁸ is OH and R⁹ is H.

In still other embodiments of any of the foregoing embodiments, each of R⁴ and R⁵ is H. In other embodiments, R⁴ is F, and in other embodiments R⁴ is OH. In still other embodiments, at least two of R¹, R², R³, R⁴ and R⁵ are F. In some embodiments, at least three of R¹, R², R³, R⁴ and R⁵ are F. In yet other embodiments, at least four of R¹, R², R³, R⁴ and R⁵ are F, and in other embodiments each of R¹, R², R³, R⁴ and R⁵ is F.

In some embodiments of any of the foregoing embodiments, at least one of R¹⁰ or R¹¹ is F, for example in some embodiments each of R¹⁰ and R¹¹ is F. In some other embodiments, at least one of of R¹⁰ or R¹¹ is methyl, for example in some embodiments each of R¹⁰ and R¹¹ is methyl.

In certain embodiments of any of the foregoing embodiments, Y is 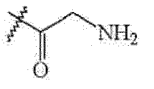 or

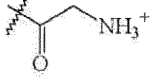

In still other embodiments, the compound of Formula (I) has one of the following structures: 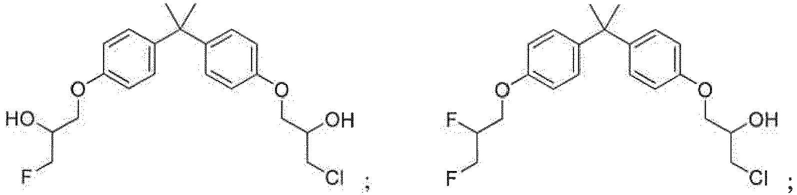 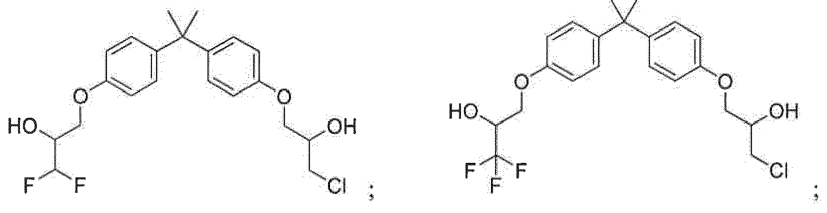 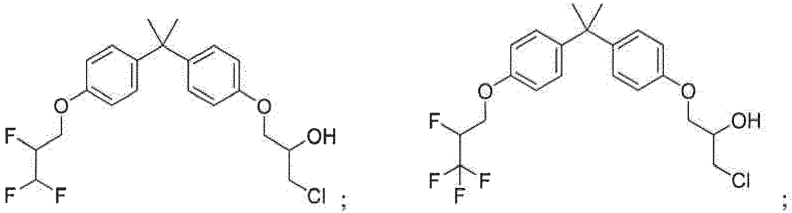 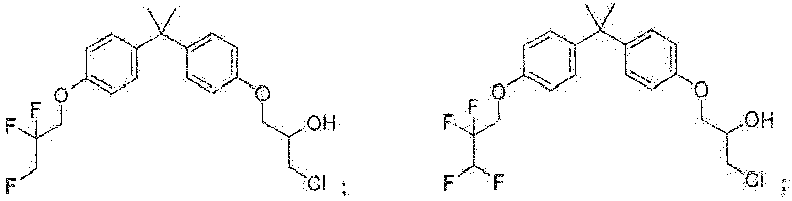 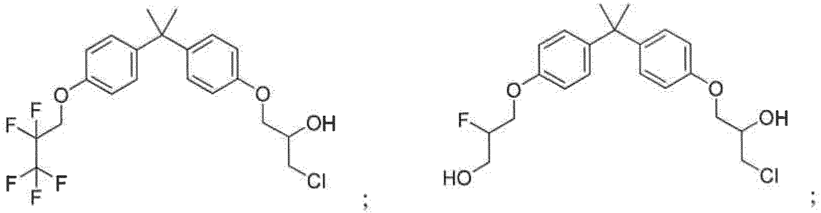 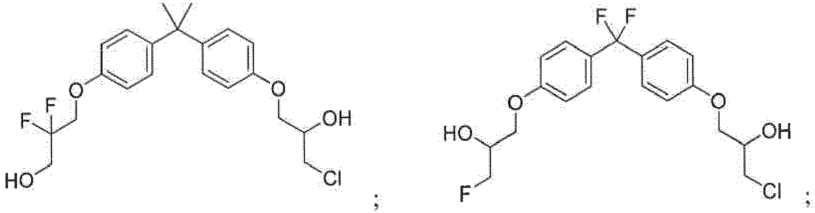 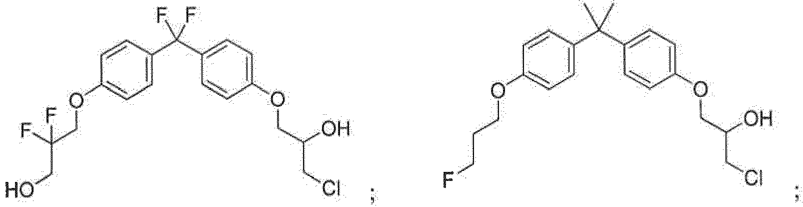 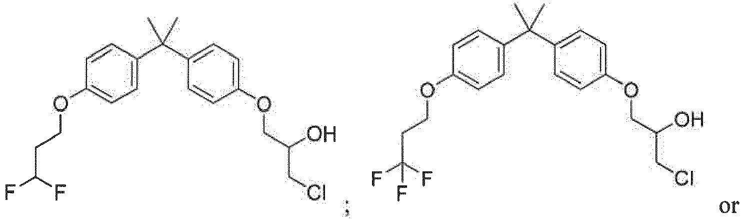 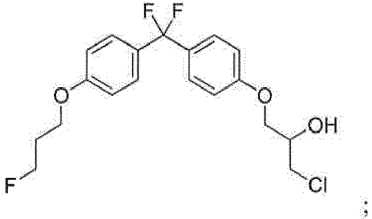 or a pharmaceutically acceptable salt or stereoisomer thereof.

In some embodiments, the compound of formula I has the following structure (1): 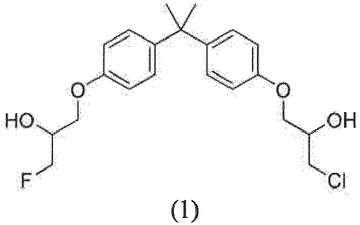 or a pharmaceutically acceptable salt or stereoisomer thereof.

In other embodiments, the compound of formula 1 has the following structure (1a): 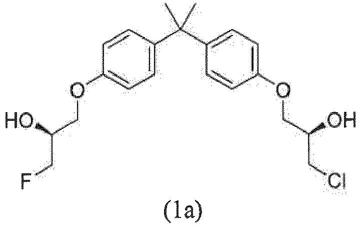 or a pharmaceutically acceptable salt thereof.

In still other embodiments, the compound of formula 1 has the following structure (1b): 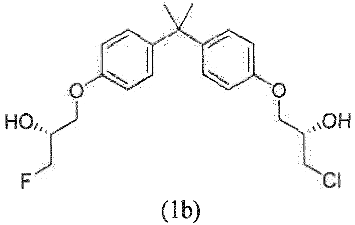 or a pharmaceutically acceptable salt thereof.

In yet other embodiments, the compound of formula 1 has the following structure (1c): 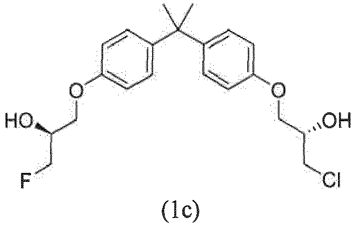 or a pharmaceutically acceptable salt thereof.

In some more embodiments, the compound of formula 1 has the following structure (1d): 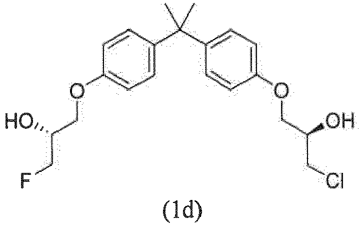 or a pharmaceutically acceptable salt thereof.Each R¹⁰ may independently be C₁-C₅ alkyl. Each R¹⁰ may independently be C₁-C₄ alkyl. Each R¹⁰ may independently be C₁-C₃ alkyl. Each R¹⁰ may independently be C₁-C₂ alkyl. Each R¹⁰ may independently be methyl. Each R¹⁰ may independently be C₂ alkyl. Each R¹⁰ may independently be C₃ alkyl. Each R¹⁰ may independently be C₄ alkyl. Each R¹⁰ may independently be C₅ alkyl. Each R¹⁰ may independently be F.

Each R¹¹ may independently be C₁-C₅ alkyl. Each R¹¹ may independently be C₁-C₄ alkyl. Each R¹¹ may independently be C₁-C₃ alkyl. Each R¹¹ may independently be C₁-C₂ alkyl. Each R¹¹ may independently be methyl. Each R¹¹ may independently be C₂ alkyl. Each R¹¹ may independently be C₃ alkyl. Each R¹¹ may independently be C₄ alkyl. Each R¹¹ may independently be C₅ alkyl. Each R¹¹ may independently be F.

In another embodiment, the present disclosure provides the use of any one of the foregoing compounds of Formula (I) for modulating androgen receptor (AR) activity. For example in some embodiments, modulating androgen receptor (AR) activity is in a mammalian cell.

In other embodiments, modulating androgen receptor (AR) activity is for treatment of at least one indication selected from the group consisting of: prostate cancer, breast cancer, ovarian cancer, endometrial cancer, salivary gland carcinoma, hair loss, acne, hirsutism, ovarian cysts, polycystic ovary disease, precocious puberty, spinal and bulbar muscular atrophy (i.e., Kennedy's disease), and age-related macular degeneration. For example in some embodiments, the indication is prostate cancer. In other embodiments, the prostate cancer is castration resistant prostate cancer. While in other embodiments, the prostate cancer is androgen-dependent prostate cancer.

In other embodiments, the present disclosure provides a method of modulating androgen receptor (AR) activity, the method comprising administering any one of the foregoing compounds of Formula (I), or pharmaceutically acceptable salt thereof to a subject in need thereof.

In other further embodiments of the foregoing method, modulating androgen receptor (AR) activity is for the treatment of one or more of the following: prostate cancer, breast cancer, ovarian cancer, endometrial cancer, salivary gland carcinoma, hair loss, acne, hirsutism, ovarian cysts, polycystic ovary disease, precocious puberty, spinal and bulbar muscular atrophy, and age-related macular degeneration. For example in some embodiments, the prostate cancer is castration resistant prostate cancer. In other embodiments, the prostate cancer is androgen-dependent prostate cancer.

In some other embodiments, the present disclosure provides a pharmaceutical composition comprising any one of the foregoing compounds of Formula (I) and a pharmaceutically acceptable carrier.

In yet another embodiment, the present disclosure provides a pharmaceutical composition comprising any one of the foregoing compounds of Formula (I), an additional therapeutic agent and a pharmaceutically acceptable carrier. For example, in some embodiments, the additional therapeutic agent is for treating prostate cancer, breast cancer, ovarian cancer, endometrial cancer, salivary gland carcinoma, hair loss, acne, hirsutism, ovarian cysts, polycystic ovary disease, precocious puberty, spinal and bulbar muscular atrophy or age-related macular degeneration.

In other embodiments, the additional therapeutic agent is MDV3100 , Orteronel (TAK-700), TOK 001; ARN-509; abiraterone acetate, bicalutamide, nilutamide, flutamide, cyproterone acetate, docetaxel, Bevacizumab (Avastin), OSU-HDAC42, VITAXIN, sunitumib, ZD-4054, Cabazitaxel (XRP-6258), MDX-010 (Ipilimumab), OGX 427, OGX 011, finasteride, dutasteride, turosteride, bexlosteride, izonsteride, FCE 28260, SKF105,111 or a related compound thereof.

In another embodiment, the present disclosure provides the use of any one of the foregoing pharmaceutical compositions for modulating androgen receptor (AR) activity. For example in some embodiments, modulating androgen receptor (AR) activity is in a mammalian cell.

In other embodiments, modulating androgen receptor (AR) activity is for treatment of at least one indication selected from the group consisting of: prostate cancer, breast cancer, ovarian cancer, endometrial cancer, salivary gland carcinoma, hair loss, acne, hirsutism, ovarian cysts, polycystic ovary disease, precocious puberty, spinal and bulbar muscular atrophy, and age-related macular degeneration. For example in some embodiments, the indication is prostate cancer. For example, in some embodiments, the prostate cancer is castration resistant prostate cancer, and in other embodiments the prostate cancer is androgen-dependent prostate cancer.

In yet another embodiment, the present disclosure provides a method of modulating androgen receptor (AR) activity, the method comprising administering any one of the foregoing pharmaceutical compositions to a subject in need thereof. For example in some embodiments, modulating androgen receptor (AR) activity is for the treatment of one or more of the following: prostate cancer, breast cancer, ovarian cancer, endometrial cancer, salivary gland carcinoma, hair loss, acne, hirsutism, ovarian cysts, polycystic ovary disease, precocious puberty, spinal and bulbar muscular atrophy, and age-related macular degeneration. In still other embodiments, the indication is prostate cancer. For example, in some embodiments, the prostate cancer is castration resistant prostate cancer, while in other embodiments, the prostate cancer is androgen-dependent prostate cancer.

The compounds described herein are meant to include all racemic mixtures and all individual enantiomers or combinations thereof, whether or not they are specifically depicted herein. Alternatively, one or more of the OH groups on the above compounds may be substituted to replace the H with a moiety selected from Table 1 (*i.e.,* to form a OY moiety).

In yet other embodiments, the present disclosure provide the use of any of the compounds disclosed herein for modulating androgen receptor (AR) activity. For example, in certain embodiments modulating androgen receptor (AR) activity is in a mammalian cell.

In other examples, modulating androgen receptor (AR) activity is for treatment of at least one indication selected from the group consisting of: prostate cancer, breast cancer, ovarian cancer, endometrial cancer, salivary gland carcinoma, hair loss, acne, hirsutism, ovarian cysts, polycystic ovary disease, precocious puberty, spinal and bulbar muscular atrophy, and age-related macular degeneration. For example, in certain embodiments the indication is prostate cancer, for example, castration resistant prostate cancer. In other examples, the prostate cancer is androgen-dependent prostate cancer.

The present disclosure also provides a method of modulating androgen receptor (AR) activity, the method comprising administering any of the compounds disclosed herein, or pharmaceutically acceptable salt thereof, to a subject in need thereof. For example, in certain specific embodiments modulating androgen receptor (AR) activity is for the treatment of one or more of the following: prostate cancer, breast cancer, ovarian cancer, endometrial cancer, salivary gland carcinoma, hair loss, acne, hirsutism, ovarian cysts, polycystic ovary disease, precocious puberty, spinal and bulbar muscular atrophy, and age-related macular degeneration

The present disclosure also provides a pharmaceutical composition comprising any one or more of the compounds disclosed herein and a pharmaceutically acceptable carrier. The pharmaceutical composition may be for treating one or more of the following: prostate cancer, breast cancer, ovarian cancer, endometrial cancer, salivary gland carcinoma, hair loss, acne, hirsutism, ovarian cysts, polycystic ovary disease, precocious puberty, spinal and bulbar muscular atrophy, and age-related macular degeneration.

In accordance with another embodiment, there is provided a use of the compounds of Formula (I) as described anywhere herein for preparation of a medicament for modulating androgen receptor (AR).

In accordance with a further embodiment, there is provided a method of screening for androgen receptor modulating compounds, wherein the compounds screened are selected from the compounds as described anywhere herein.

The modulating of the androgen receptor (AR) activity may be in a mammalian cell. The modulating of the androgen receptor (AR) activity may be in a mammal. The mammal may be a human.

Alternatively, the administering may be to a mammal. The administering may be to a mammal in need thereof and in an effective amount for the treatment of at least one indication selected from the group consisting of: prostate cancer, breast cancer, ovarian cancer, endometrial cancer, salivary gland carcinoma, hair loss, acne, hirsutism, ovarian cysts, polycystic ovary disease, precocious puberty, spinal and bulbar muscular atrophy (e.g., Kennedy's disease), and age-related macular degeneration.

The mammalian cell may be a human cell. The modulating AR activity may be for inhibiting AR N-terminal domain activity. The modulating AR activity may be for inhibiting AR activity. The modulating may be in vivo. The modulating AR activity may be for treatment of at least one indication selected from the group consisting of: prostate cancer, breast cancer, ovarian cancer, endometrial cancer, salivary gland carcinoma, hair loss, acne, hirsutism, ovarian cysts, polycystic ovary disease, precocious puberty, spinal and bulbar muscular atrophy (*e.g.,* Kennedy's disease), and age-related macular degeneration. The indication may be prostate cancer. The prostate cancer may be castration-resistant prostate cancer. The prostate cancer may be androgen-dependent prostate cancer.

**Table 1**

| Amino Acid. Polyethylene Glycol, and Phosphate Based Moieties | |
|---|---|
| Amino Acid Based Moieties | |
| 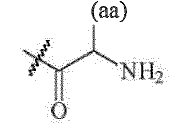 | 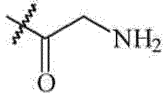 |
| (aa) = any naturally occurring amino acid side chain | |
| 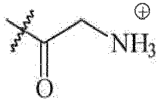 | 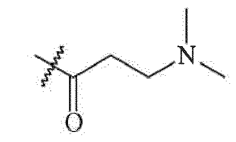 |
| Polyethylene Glycol Based Moieties | |
| 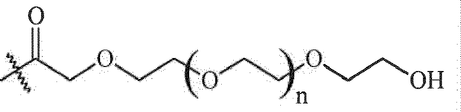 | 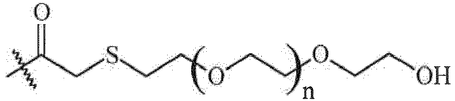 |
| n = 1-200 | n= 1-200 |
| Phosphate Based Moieties | |
| 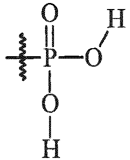 | 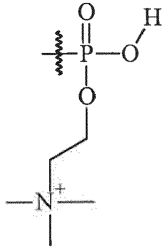 |
| 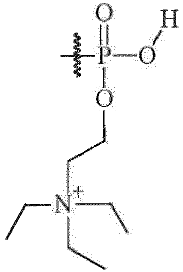 | 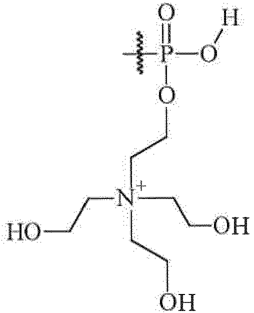 |
| 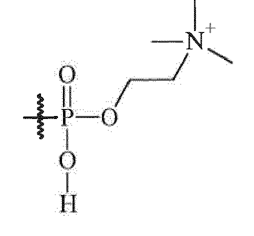 | 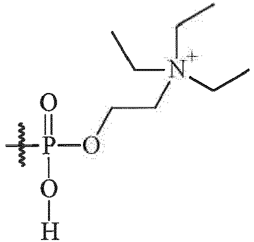 |
| 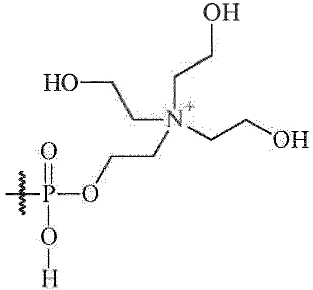 | 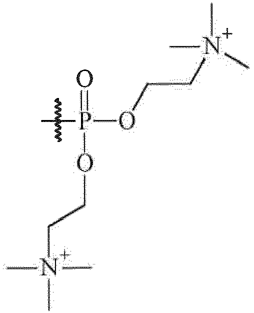 |
| 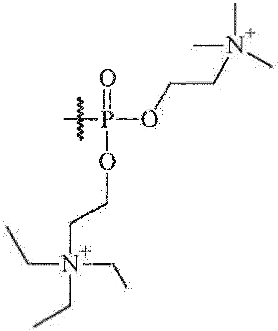 | 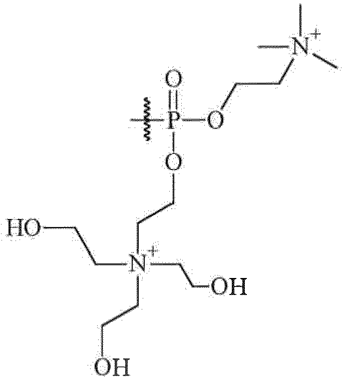 |
| 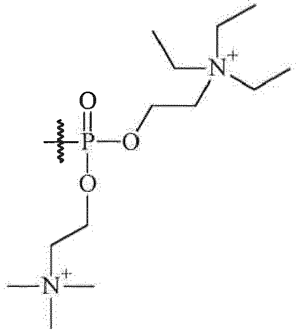 | 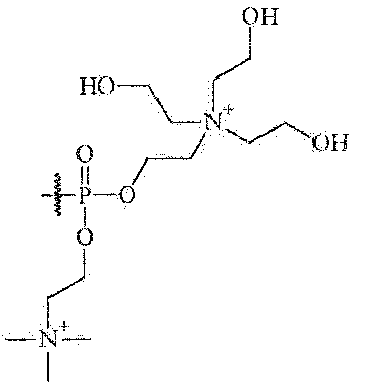 |
| 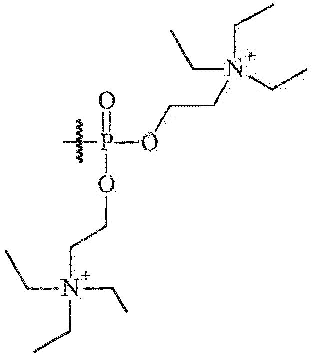 | 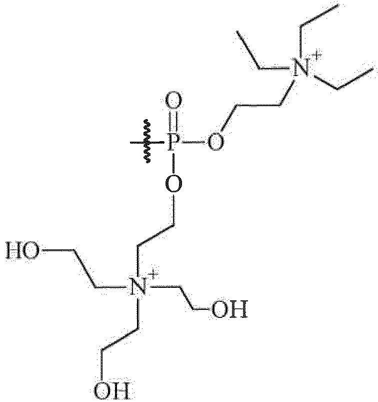 |
| 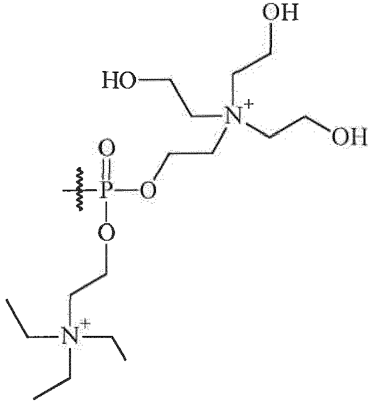 | 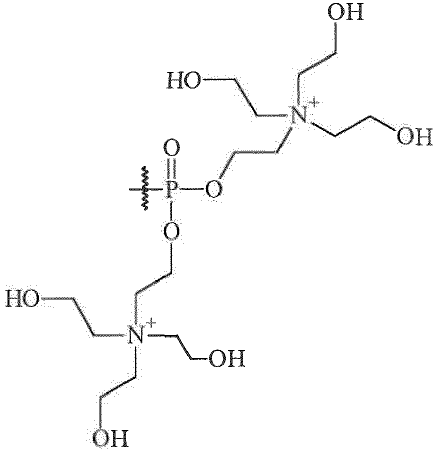 |

Moieties from TABLE 1 may be, for example, and without limitation, subdivided into three groups: 1) amino acid based moieties; 2) polyethylene glycol based moieties; and 3) phosphate based moieties. In the Moieties Table 1 above, the first four moieties are amino acid based moieties, the fifth and sixth are polyethylene glycol based moieties and the remaining moieties are phosphate based moieties.

The amino acid side chains of naturally occurring amino acids (as often denoted herein using "(aa)") are well known to a person of skill in the art and may be found in a variety of text books such as "Molecular Cell Biology" by James Darnell et al. Third Edition, published by Scientific American Books in 1995. Often the naturally occurring amino acids are represented by the formula (NH₂)C(COOH)(H)(R), where the chemical groups in brackets are each bonded to the carbon not in brackets. R represents the side chains in this particular formula.

Those skilled in the art will appreciate that the point of covalent attachment of the moiety to the compounds as described herein may be, for example, and without limitation, cleaved under specified conditions. Specified conditions may include, for example, and without limitation, *in vivo* enzymatic or non-enzymatic means. Cleavage of the moiety may occur, for example, and without limitation, spontaneously, or it may be catalyzed, induced by another agent, or a change in a physical parameter or environmental parameter, for example, an enzyme, light, acid, temperature or pH. The moiety may be, for example, and without limitation, a protecting group that acts to mask a functional group, a group that acts as a substrate for one or more active or passive transport mechanisms, or a group that acts to impart or enhance a property of the compound, for example, solubility, bioavailability or localization.

In other particular embodiments of the compounds as described anywhere herein, the compound is a compound selected from one of the compounds in Table 2.

**Table 2**

| Representative Compounds | | |
|---|---|---|
| No. | Structure | Name |
| 1 | 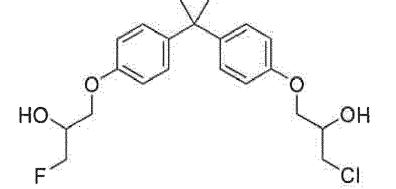 | 1-chloro-3-(4-(2-(4-(3-fluoro-2-hydroxypropoxy)phenyl)propan-2-yl)phenoxy)propan-2-ol |
| 1a | 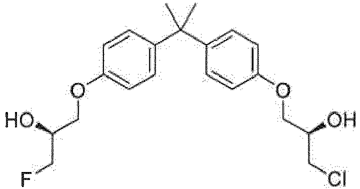 | (R)-1-chloro-3-(4-(2-(4-((S)-3-fluoro-2-hydroxypropoxy)phenyl)propan-2-yl)phenoxy)propan-2-ol |
| 1b | 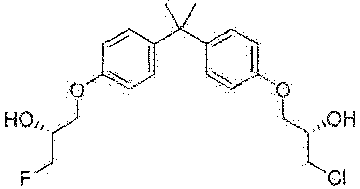 | (S)-1-chloro-3-(4-(2-(4-((R)-3-fluoro-2-hydroxypropoxy)phenyl)propan-2-yl)phenoxy)propan-2-ol |
| 1c | 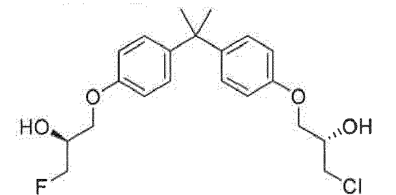 | (S)-1-chloro-3-(4-(2-(4-((S)-3-fluoro-2-hydroxypropoxy)phenyl)propan-2-yl)phenoxy)propan-2-ol |

| No. | Structure | Name |
|---|---|---|
| 1d | 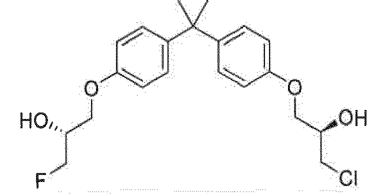 | (R)-1-chloro-3-(4-(2-(4-((R)-3-fluoro-2-hydroxypropoxy)phenyl)propan-2-yl)phenoxy)propan-2-ol |
| 2 | 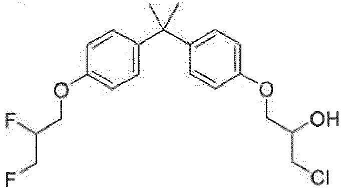 | 1-chloro-3-(4-(2-(4-(2,3-difluoropropoxy)phenyl)propan-2-yl)phenoxy)propan-2-ol |
| 2a | 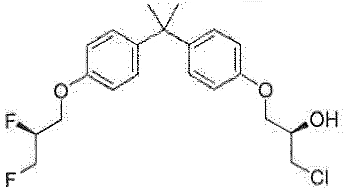 | (R)-1-chloro-3-(4-(2-(4-((S)-2,3-difluoropropoxy)phenyl)propan-2-yl)phenoxy)propan-2-ol |
| 2b | 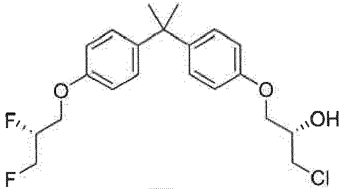 | (S)-1-chloro-3-(4-(2-(4-((R)-2,3-difluoropropoxy)phenyl)propan-2-yl)phenoxy)propan-2-ol |
| 2c | 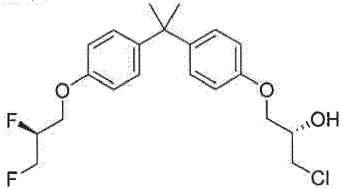 | (S)-1-chloro-3-(4-(2-(4-((S)-2,3-difluoropropoxy)phenyl)propan-2-yl)phenoxy)propan-2-ol |
| 2d | 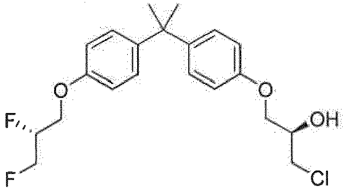 | (R)-1-chloro-3-(4-(2-(4-((R)-2,3-difluoropropoxy)phenyl)propan-2-yl)phenoxy)propan-2-ol |
| 3 | 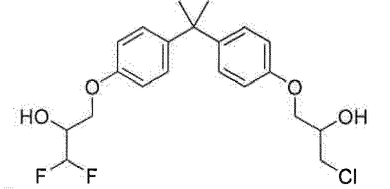 | 3-(4-(2-(4-(3-chloro-2-hydroxypropoxy)phenyl)propan-2-yl) phenoxy)-1,1-difluoropropan-2-ol |
| 3a | 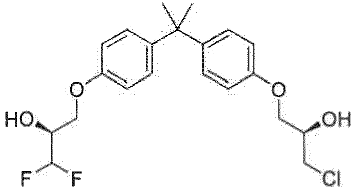 | (S)-3-(4-(2-(4-((R)-3-chloro-2-hydroxypropoxy)phenyl)propan-2-yl) phenoxy)-1,1-difluoropropan-2-ol |
| 3b | 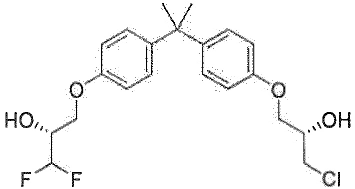 | (R)-3-(4-(2-(4-((S)-3-chloro-2-hydroxypropoxy)phenyl)propan-2-yl) phenoxy)-1,1-difluoropropan-2-ol |
| 3c | 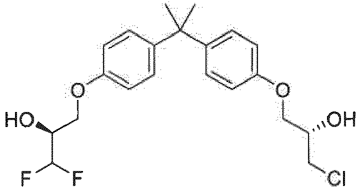 | (S)-3-(4-(2-(4-((S)-3-chloro-2-hydroxypropoxy)phenyl)propan-2-yl) phenoxy)-1,1-difluoropropan-2-ol |
| 3d | 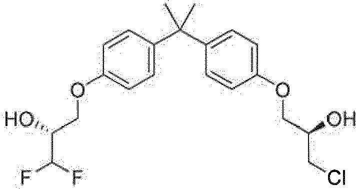 | (R)-3-(4-(2-(4-((R)-3-chloro-2-hydroxypropoxy)phenyl)propan-2-yl) phenoxy)-1,1-difluoropropan-2-ol |
| 4 | 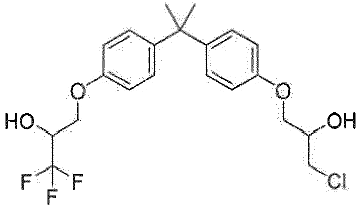 | 3-(4-(2-(4-(3-chloro-2-hydroxypropoxy)phenyl)propan-2-yl) phenoxy)-1,1,1-trifluoropropan-2-ol |
| 4a | 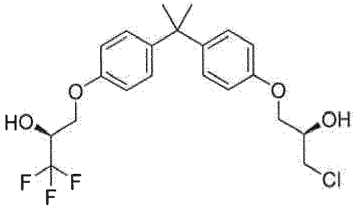 | (S)-3-(4-(2-(4-((R)-3-chloro-2-hydroxypropoxy)phenyl)propan-2-yl) phenoxy)-1,1,1-trifluoropropan-2-ol |
| 4b | 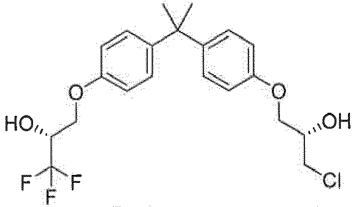 | (R)-3-(4-(2-(4-((S)-3-chloro-2-hydroxypropoxy)phenyl)propan-2-yl) phenoxy)-1,1,1-trifluoropropan-2-ol |
| 4c | 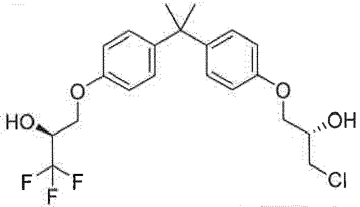 | (S)-3-(4-(2-(4-((S)-3-chloro-2-hydroxypropoxy)phenyl)propan-2-yl) phenoxy)-1,1,1-trifluoropropan-2-ol |
| 4d | 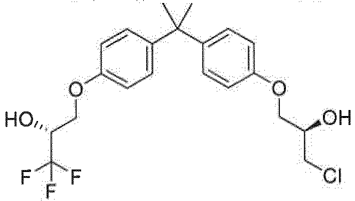 | (R)-3-(4-(2-(4-((R)-3-chloro-2-hydroxypropoxy)phenyl)propan-2-yl) phenoxy)-1,1,1-trifluoropropan-2-ol |
| 5 | 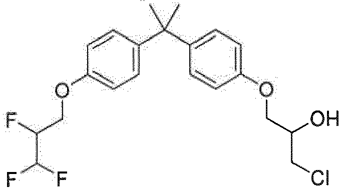 | 1-chloro-3-(4-(2-(4-(2,3,3-trifluoropropoxy)phenyl)propan-2-yl)phenoxy)propan-2-ol |
| 5a | 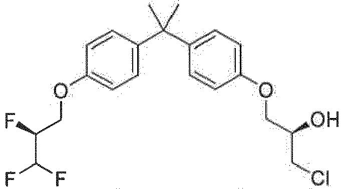 | (R)-1-chloro-3-(4-(2-(4-((S)-2,3,3-trifluoropropoxy)phenyl)propan-2-yl)phenoxy)propan-2-ol |
| 5b | 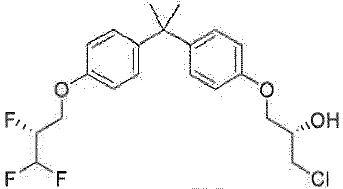 | (S)-1-chloro-3-(4-(2-(4-((R)-2,3,3-trifluoropropoxy)phenyl)propan-2-yl)phenoxy)propan-2-ol |
| 5c | 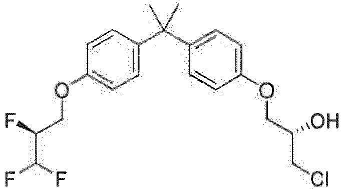 | (S)-1-chloro-3-(4-(2-(4-((S)-2,3,3-trifluoropropoxy)phenyl)propan-2-yl)phenoxy)propan-2-ol |
| 5d | 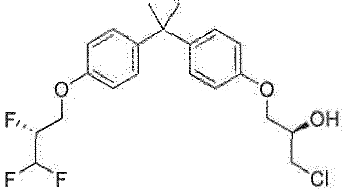 | (R)-1-chloro-3-(4-(2-(4-((R)-2,3,3-trifluoropropoxy)phenyl)propan-2-yl)phenoxy)propan-2-ol |
| 6 | 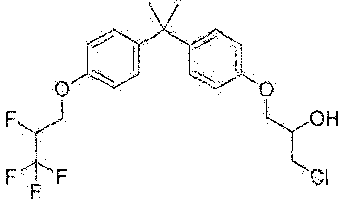 | 1-chloro-3-(4-(2-(4-(2,3,3,3-tetrafluoropropoxy)phenyl)propan-2-yl)phenoxy)propan-2-ol |
| 6a | 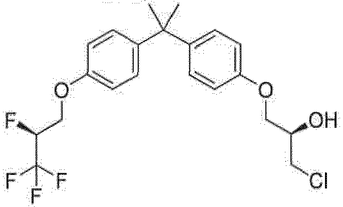 | (R)-1-chloro-3-(4-(2-(4-((S)-2,3,3,3-tetrafluoropropoxy)phenyl)propan-2-yl)phenoxy)propan-2-ol |
| 6b | 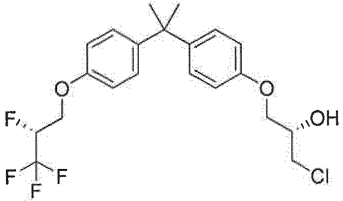 | (S)-1-chloro-3-(4-(2-(4-((R)-2,3,3,3-tetrafluoropropoxy)phenyl)propan-2-yl)phenoxy)propan-2-ol |
| 6c | 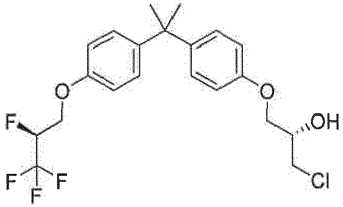 | (S)-1-chloro-3-(4-(2-(4-((S)-2,3,3,3-tetrafluoropropoxy)phenyl)propan-2-yl)phenoxy)propan-2-ol |
| 6d | 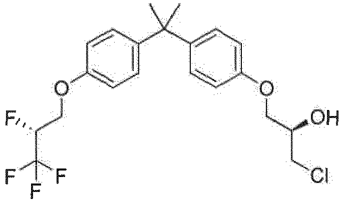 | (R)-1-chloro-3-(4-(2-(4-((R)-2,3,3,3-tetrafluoropropoxy)phenyl)propan-2-yl)phenoxy)propan-2-ol |
| 7 | 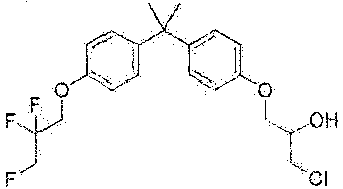 | 1-chloro-3-(4-(2-(4-(3-fluoro-2-hydroxypropoxy)phenyl)propan-2-yl)phenoxy)propan-2-ol |
| 7a | 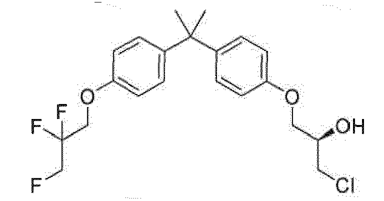 | (R)-1-chloro-3-(4-(2-(4-(2,2,3-trifluoropropoxy)phenyl)propan-2-yl)phenoxy)propan-2-ol |
| 7b | 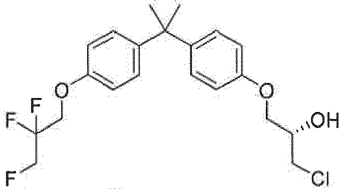 | (S)-1-chloro-3-(4-(2-(4-(2,2,3-trifluoropropoxy)phenyl)propan-2-yl)phenoxy)propan-2-ol |
| 8 | 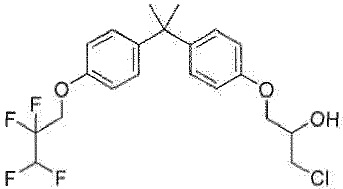 | 1-chloro-3-(4-(2-(4-(2,2,3,3-tetrafluoropropoxy)phenyl)propan-2-yl)phenoxy)propan-2-ol |
| 8a | 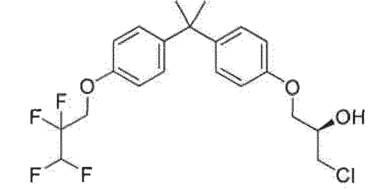 | (R)-1-chloro-3-(4-(2-(4-(2,2,3,3-tetrafluoropropoxy)phenyl)propan-2-yl)phenoxy)propan-2-ol |
| 8b | 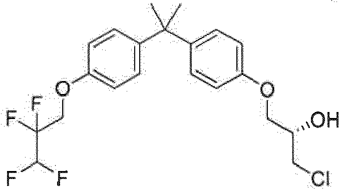 | (S)-1-chloro-3-(4-(2-(4-(2,2,3,3-tetrafluoropropoxy)phenyl)propan-2-yl)phenoxy)propan-2-ol |
| 9 | 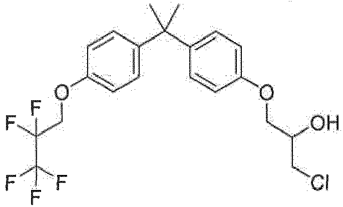 | 1-chloro-3-(4-(2-(4-(2,2,3,3,3-pentafluoropropoxy)phenyl)propan-2-yl)phenoxy)propan-2-ol |
| 9a | 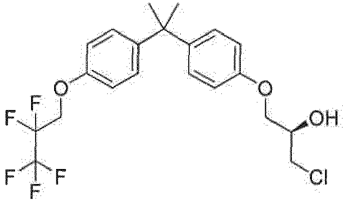 | (R)-1-chloro-3-(4-(2-(4-(2,2,3,3,3-pentafluoropropoxy)phenyl)propan-2-yl)phenoxy)propan-2-ol |
| 9b | 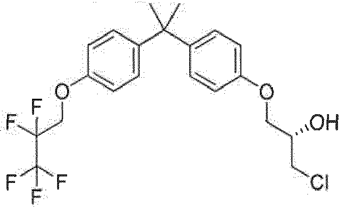 | (S)-1-chloro-3-(4-(2-(4-(2,2,3,3,3-pentafluoropropoxy)phenyl)propan-2-yl)phenoxy)propan-2-ol |
| 10 | 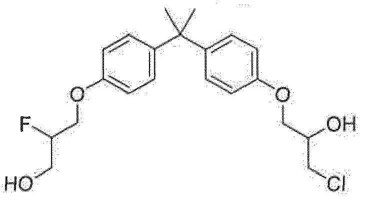 | 3-(4-(2-(4-(3-chloro-2-hydroxypropoxy)phenyl)propan-2-yl) phenoxy)-2-fluoropropan-1-ol |
| 10a | 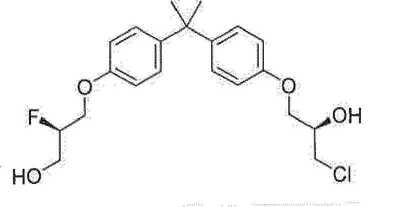 | (R)-3-(4-(2-(4-((R)-3-chloro-2-hydroxypropoxy)phenyl)propan-2-yl) phenoxy)-2-fluoropropan-1-ol |
| 10b | 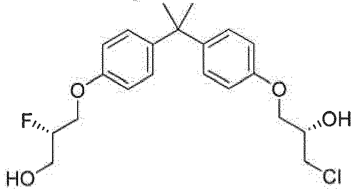 | (S)-3-(4-(2-(4-((S)-3-chloro-2-hydroxypropoxy)phenyl)propan-2-yl) phenoxy)-2-fluoropropan-1-ol |
| 10c | 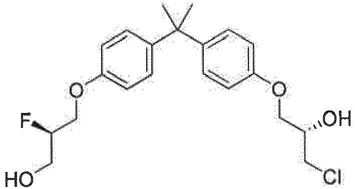 | (R)-3-(4-(2-(4-((S)-3-chloro-2-hydroxypropoxy)phenyl)propan-2-yl) phenoxy)-2-fluoropropan-1-ol |
| 10d | 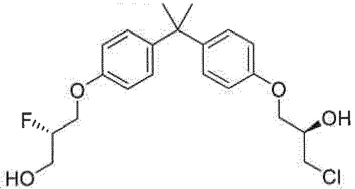 | (S)-3-(4-(2-(4-((R)-3-chloro-2-hydroxypropoxy)phenyl)propan-2-yl) phenoxy)-2-fluoropropan-1-ol |
| 11 | 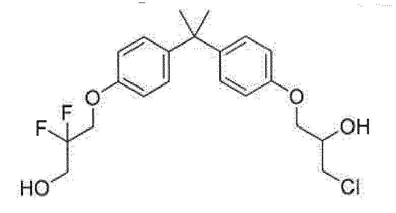 | 3-(4-(2-(4-(3-chloro-2-hydroxypropoxy)phenyl)propan-2-yl) phenoxy)-2,2-difluoropropan-1-ol |
| 11a | 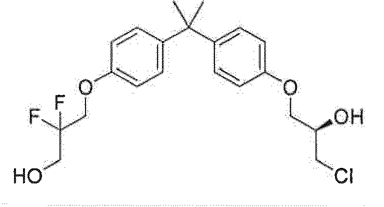 | (R)-3-(4-(2-(4-(3-chloro-2-hydroxypropoxy)phenyl)propan-2-yl) phenoxy)-2,2-difluoropropan-1-ol |
| 11b | 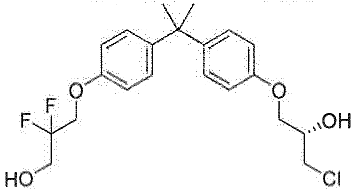 | (S)-3-(4-(2-(4-(3-chloro-2-hydroxypropoxy)phenyl)propan-2-yl) phenoxy)-2,2-difluoropropan-1-ol |
| 12 | 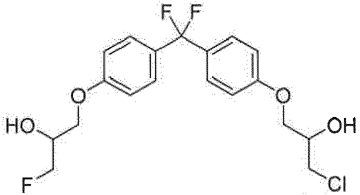 | 1-chloro-3-(4-(difluoro(4-(3-fluoro-2-hydroxypropoxy)phenyl) methyl)phenoxy)propan-2-ol |
| 12a | 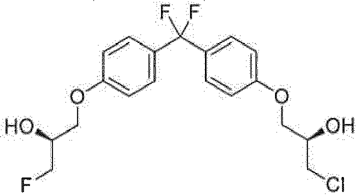 | (R)-1-chloro-3-(4-(difluoro(4-((S)-3-fluoro-2hydroxypropoxy)phenyl)methyl) phenoxy)propan-2-ol |
| 12b | 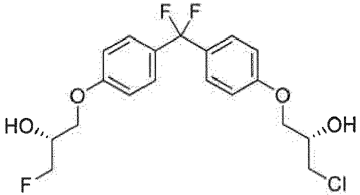 | (S)-1-chloro-3-(4-(difluoro(4-((R)-3-fluoro-2-hydroxypropoxy)phenyl)methyl) phenoxy)propan-2-ol |
| 12c | 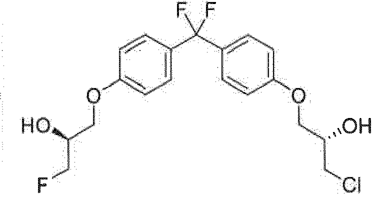 | (S)-1-chloro-3-(4-(difluoro(4-((S)-3-fluoro-2-hydroxypropoxy)phenyl)methyl) phenoxy)propan-2-ol |
| 12d | 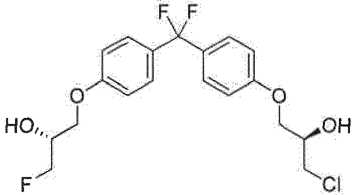 | (R)-1-chloro-3-(4-(difluoro(4-((R)-3-fluoro-2-hydroxypropoxy)phenyl)methyl) phenoxy)propan-2-ol |
| 13 | 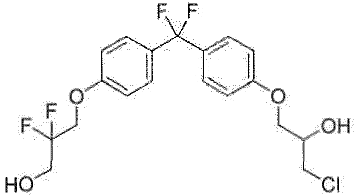 | 3-(4-((4-(3-chloro-2-hydroxypropoxy)phenyl)difluoromethyl) phenoxy)-2,2-difluoropropan-1-ol |
| 13a | 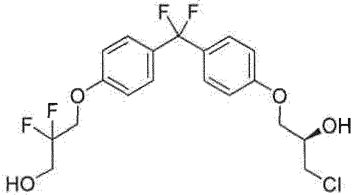 | (R)-3-(4-((4-(3-chloro-2-hydroxypropoxy)phenyl)difluoromethyl) phenoxy)-2,2-difluoropropan-1-ol |
| 13b | 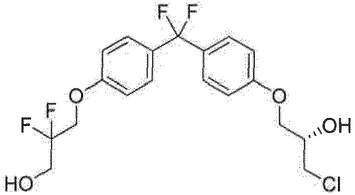 | (S)-3-(4-((4-(3-chloro-2-hydroxypropoxy)phenyl)difluoromethyl) phenoxy)-2,2-difluoropropan-1-ol |
| 14 | 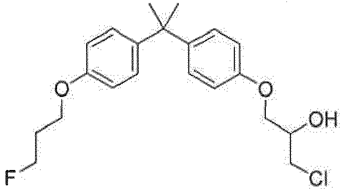 | 1-chloro-3-(4-(2-(4-(3-fluoropropoxy)phenyl)propan-2-yl)phenoxy)propan-2-ol |
| 14a | 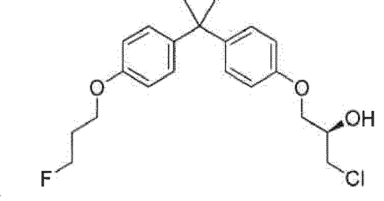 | (R)-1-chloro-3-(4-(2-(4-(3-fluoropropoxy)phenyl)propan-2-yl)phenoxy)propan-2-ol |
| 14b | 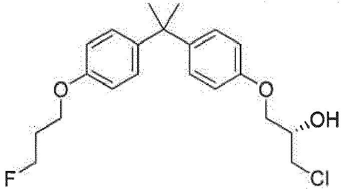 | (S)-1-chloro-3-(4-(2-(4-(3-fluoropropoxy)phenyl)propan-2-yl)phenoxy)propan-2-ol |
| 15 | 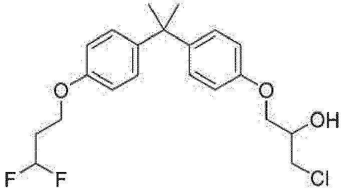 | 1-chloro-3-(4-(2-(4-(3,3-difluoropropoxy)phenyl)propan-2-yl)phenoxy)propan-2-ol |
| 15a | 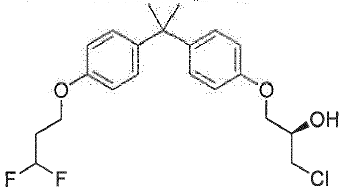 | (R)-1-chloro-3-(4-(2-(4-(3,3-difluoropropoxy)phenyl)propan-2-yl)phenoxy)propan-2-ol |
| 15b | 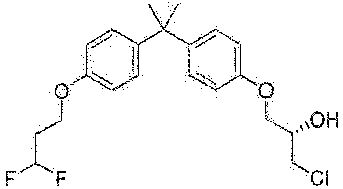 | (S)-1-chloro-3-(4-(2-(4-(3,3-difluoropropoxy)phenyl)propan-2-yl)phenoxy)propan-2-ol |
| 16 | 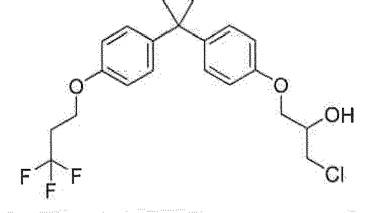 | 1-chloro-3-(4-(2-(4-(3,3,3-trifluoropropoxy)phenyl)propan-2-yl)phenoxy)propan-2-ol |
| 16a | 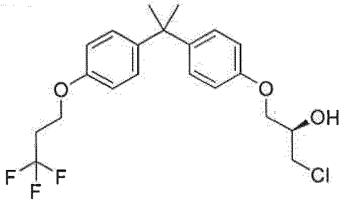 | (R)-1-chloro-3-(4-(2-(4-(3,3,3-trifluoropropoxy)phenyl)propan-2-yl)phenoxy)propan-2-ol |
| 16b | 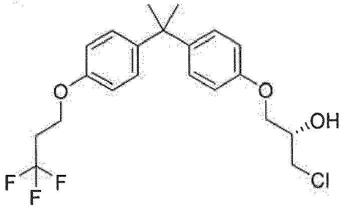 | (S)-1-chloro-3-(4-(2-(4-(3,3,3-trifluoropropoxy)phenyl)propan-2-yl)phenoxy)propan-2-ol |
| 17 | 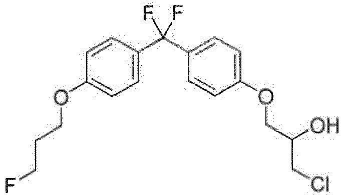 | 1-chloro-3-(4-(difluoro(4-(3-fluoropropoxy)phenyl)methyl)phenoxy) propan-2-ol |
| 17a | 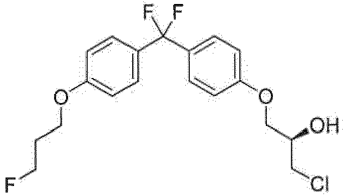 | (R)-1-chloro-3-(4-(difluoro(4-(3-fluoropropoxy)phenyl)methyl)phenoxy) propan-2-ol |
| 17b | 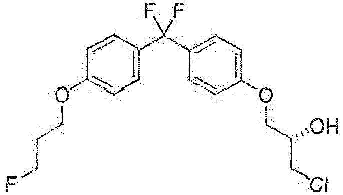 | (S)-1-chloro-3-(4-(difluoro(4-(3-fluoropropoxy)phenyl)methyl)phenoxy) propan-2-ol |

Prodrugs are also included within the scope of the present disclosure. For example, in one embodiment the hydrogen atom of one or more hydroxyl groups of any of the compounds of Formula I may be replaced with a moiety from Table 1 (*i.e*., to form a OY moiety). Non-limiting examples of such prodrugs include glycine esters and salts thereof as shown below.

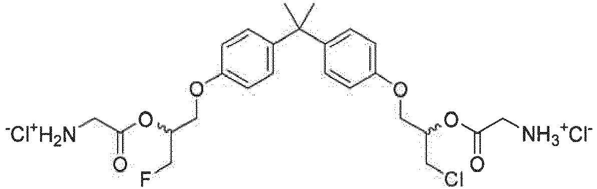

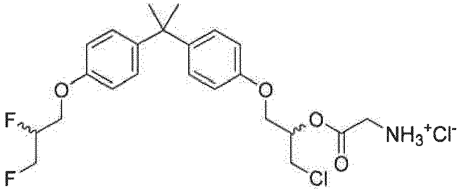

In some embodiments, the compounds as described herein or acceptable salts thereof may be used for systemic treatment of at least one indication selected from the group consisting of: prostate cancer, breast cancer, ovarian cancer, endometrial cancer, salivary gland carcinoma, hair loss, acne, hirsutism, ovarian cysts, polycystic ovary disease, precocious puberty, spinal and bulbar muscular atrophy, and age-related macular degeneration. In some embodiments, the compounds as described herein or acceptable salts thereof above may be used in the preparation of a medicament or a composition for systemic treatment of an indication described herein. In some embodiments, methods of systemically treating any of the indications described herein are also provided. Some aspects of this disclosure, make use of compositions comprising a compound described herein and a pharmaceutically acceptable excipients or carrier. In some embodiments, the prostate cancer is castration-resistant prostate cancer (also referred to as hormone refractory, androgen-independent, androgen deprivation resistant, androgen ablation resistant, androgen depletion-independent, castration-recurrent, anti-androgen-recurrent). In some embodiments the prostate cancer is androgen-dependent or androgen-sensitive. Methods of treating any of the indications described herein are also provided. Such methods may include administering a compound as described herein or a composition of a compound as described herein, or an effective amount of a compound as described herein or composition of a compound as described herein to a subject in need thereof.

Compounds as described herein include all stereoisomers. Accordingly, the compounds include racemic mixtures, enantiomers and diastereomers of any of the compounds described herein.

Compounds as described herein may be in the free form or in the form of a salt thereof. In some embodiments, compounds as described herein may be in the form of a pharmaceutically acceptable salt, which are known in the art (Berge et al., J. Pharm. Sci. 1977, 66, 1). Pharmaceutically acceptable salt as used herein includes, for example, salts that have the desired pharmacological activity of the parent compound (salts which retain the biological effectiveness and/or properties of the parent compound and which are not biologically and/or otherwise undesirable). Compounds as described herein having one or more functional groups capable of forming a salt may be, for example, formed as a pharmaceutically acceptable salt. Compounds containing one or more basic functional groups may be capable of forming a pharmaceutically acceptable salt with, for example, a pharmaceutically acceptable organic or inorganic acid. Pharmaceutically acceptable salts may be derived from, for example, and without limitation, acetic acid, adipic acid, alginic acid, aspartic acid, ascorbic acid, benzoic acid, benzenesulfonic acid, butyric acid, cinnamic acid, citric acid, camphoric acid, camphorsulfonic acid, cyclopentanepropionic acid, diethylacetic acid, digluconic acid, dodecylsulfonic acid, ethanesulfonic acid, formic acid, fumaric acid, glucoheptanoic acid, gluconic acid, glycerophosphoric acid, glycolic acid, hemisulfonic acid, heptanoic acid, hexanoic acid, hydrochloric acid, hydrobromic acid, hydriodic acid, 2-hydroxyethanesulfonic acid, isonicotinic acid, lactic acid, malic acid, maleic acid, malonic acid, mandelic acid, methanesulfonic acid, 2-napthalenesulfonic acid, naphthalenedisulphonic acid, p-toluenesulfonic acid, nicotinic acid, nitric acid, oxalic acid, pamoic acid, pectinic acid, 3-phenylpropionic acid, phosphoric acid, picric acid, pimelic acid, pivalic acid, propionic acid, pyruvic acid, salicylic acid, succinic acid, sulfuric acid, sulfamic acid, tartaric acid, thiocyanic acid or undecanoic acid. Compounds containing one or more acidic functional groups may be capable of forming pharmaceutically acceptable salts with a pharmaceutically acceptable base, for example, and without limitation, inorganic bases based on alkaline metals or alkaline earth metals or organic bases such as primary amine compounds, secondary amine compounds, tertiary amine compounds, quaternary amine compounds, substituted amines, naturally occurring substituted amines, cyclic amines or basic ion-exchange resins. Pharmaceutically acceptable salts may be derived from, for example, and without limitation, a hydroxide, carbonate, or bicarbonate of a pharmaceutically acceptable metal cation such as ammonium, sodium, potassium, lithium, calcium, magnesium, iron, zinc, copper, manganese or aluminum, ammonia, benzathine, meglumine, methylamine, dimethylamine, trimethylamine, ethylamine, diethylamine, triethylamine, isopropylamine, tripropylamine, tributylamine, ethanolamine, diethanolamine, 2-dimethylaminoethanol, 2-diethylaminoethanol, dicyclohexylamine, lysine, arginine, histidine, caffeine, hydrabamine, choline, betaine, ethylenediamine, glucosamine, glucamine, methylglucamine, theobromine, purines, piperazine, piperidine, procaine, N-ethylpiperidine, theobromine, tetramethylammonium compounds, tetraethylammonium compounds, pyridine, N,N-dimethylaniline, N-methylpiperidine, morpholine, N-methylmorpholine, N-ethylmorpholine, dicyclohexylamine, dibenzylamine, N,N-dibenzylphenethylamine, 1-ephenamine, N,N'-dibenzylethylenediamine or polyamine resins. In some embodiments, compounds as described herein may contain both acidic and basic groups and may be in the form of inner salts or zwitterions, for example, and without limitation, betaines. Salts as described herein may be prepared by conventional processes known to a person skilled in the art, for example, and without limitation, by reacting the free form with an organic acid or inorganic acid or base, or by anion exchange or cation exchange from other salts. Those skilled in the art will appreciate that preparation of salts may occur *in situ* during isolation and purification of the compounds or preparation of salts may occur by separately reacting an isolated and purified compound.

In some embodiments, compounds and all different forms thereof (*e*.*g*., free forms, salts, polymorphs, isomeric forms) as described herein may be in the solvent addition form, for example, solvates. Solvates contain either stoichiometric or non-stoichiometric amounts of a solvent in physical association the compound or salt thereof. The solvent may be, for example, and without limitation, a pharmaceutically acceptable solvent. For example, hydrates are formed when the solvent is water or alcoholates are formed when the solvent is an alcohol.

In some embodiments, compounds and all different forms thereof *(e.g.,* free forms, salts, solvates, isomeric forms) as described herein may include crystalline and amorphous forms, for example, polymorphs, pseudopolymorphs, conformational polymorphs, amorphous forms, or a combination thereof. Polymorphs include different crystal packing arrangements of the same elemental composition of a compound. Polymorphs usually have different X-ray diffraction patterns, infrared spectra, melting points, density, hardness, crystal shape, optical and electrical properties, stability and/or solubility. Those skilled in the art will appreciate that various factors including recrystallization solvent, rate of crystallization and storage temperature may cause a single crystal form to dominate.

In some embodiments, compounds and all different forms thereof (e.g., free forms, salts, solvates, polymorphs) as described herein include isomers such as geometrical isomers, optical isomers based on asymmetric carbon, stereoisomers, tautomers, individual enantiomers, individual diastereomers, racemates, diastereomeric mixtures and combinations thereof, and are not limited by the description of the formula illustrated for the sake of convenience.

In some embodiments, pharmaceutical compositions in accordance with this invention may comprise a salt of such a compound, preferably a pharmaceutically or physiologically acceptable salt. Pharmaceutical preparations will typically comprise one or more carriers, excipients or diluents acceptable for the mode of administration of the preparation, be it by injection, inhalation, topical administration, lavage, or other modes suitable for the selected treatment. Suitable carriers, excipients or diluents are those known in the art for use in such modes of administration.

Suitable pharmaceutical compositions may be formulated by means known in the art and their mode of administration and dose determined by the skilled practitioner. For parenteral administration, a compound may be dissolved in sterile water or saline or a pharmaceutically acceptable vehicle used for administration of non-water soluble compounds such as those used for vitamin K. For enteral administration, the compound may be administered in a tablet, capsule or dissolved in liquid form. The tablet or capsule may be enteric coated, or in a formulation for sustained release. Many suitable formulations are known, including, polymeric or protein microparticles encapsulating a compound to be released, ointments, pastes, gels, hydrogels, or solutions which can be used topically or locally to administer a compound. A sustained release patch or implant may be employed to provide release over a prolonged period of time. Many techniques known to one of skill in the art are described in Remington: the Science & Practice of Pharmacy by Alfonso Gennaro, 20th ed., Lippencott Williams & Wilkins, (2000). Formulations for parenteral administration may, for example, contain excipients, polyalkylene glycols such as polyethylene glycol, oils of vegetable origin, or hydrogenated naphthalenes. Biocompatible, biodegradable lactide polymer, lactide/glycolide copolymer, or polyoxyethylene-polyoxypropylene copolymers may be used to control the release of the compounds. Other potentially useful parenteral delivery systems for modulatory compounds include ethylene-vinyl acetate copolymer particles, osmotic pumps, implantable infusion systems, and liposomes. Formulations for inhalation may contain excipients, for example, lactose, or may be aqueous solutions containing, for example, polyoxyethylene-9-lauryl ether, glycocholate and deoxycholate, or may be oily solutions for administration in the form of nasal drops, or as a gel.

Compounds or pharmaceutical compositions in accordance with this invention or for use in this invention may be administered by means of a medical device or appliance such as an implant, graft, prosthesis, stent, etc. Also, implants may be devised which are intended to contain and release such compounds or compositions. An example would be an implant made of a polymeric material adapted to release the compound over a period of time.

An "effective amount" of a pharmaceutical composition according to the invention includes a therapeutically effective amount or a prophylactically effective amount. A "therapeutically effective amount" refers to an amount effective, at dosages and for periods of time necessary, to achieve the desired therapeutic result, such as reduced tumor size, increased life span or increased life expectancy. A therapeutically effective amount of a compound may vary according to factors such as the disease state, age, sex, and weight of the subject, and the ability of the compound to elicit a desired response in the subject. Dosage regimens may be adjusted to provide the optimum therapeutic response. A therapeutically effective amount is also one in which any toxic or detrimental effects of the compound are outweighed by the therapeutically beneficial effects. A "prophylactically effective amount" refers to an amount effective, at dosages and for periods of time necessary, to achieve the desired prophylactic result, such as smaller tumors, increased life span, increased life expectancy or prevention of the progression of prostate cancer to the lethal castration resistant stage. Typically, a prophylactic dose is used in subjects prior to or at an earlier stage of disease, so that a prophylactically effective amount may be less than a therapeutically effective amount.

It is to be noted that dosage values may vary with the severity of the condition to be alleviated. For any particular subject, specific dosage regimens may be adjusted over time according to the individual need and the professional judgment of the person administering or supervising the administration of the compositions. Dosage ranges set forth herein are exemplary only and do not limit the dosage ranges that may be selected by medical practitioners. The amount of active compound(s) in the composition may vary according to factors such as the disease state, age, sex, and weight of the subject. Dosage regimens may be adjusted to provide the optimum therapeutic response. For example, a single bolus may be administered, several divided doses may be administered over time or the dose may be proportionally reduced or increased as indicated by the exigencies of the therapeutic situation. It may be advantageous to formulate parenteral compositions in dosage unit form for ease of administration and uniformity of dosage.

In some embodiments, compounds and all different forms thereof as described herein may be used, for example, and without limitation, in combination with other treatment methods for at least one indication selected from the group consisting of: prostate cancer, breast cancer, ovarian cancer, endometrial cancer, salivary gland carcinoma, hair loss, acne, hirsutism, ovarian cysts, polycystic ovary disease, precocious puberty, spinal and bulbar muscular atrophy, and age-related macular degeneration. For example, compounds and all their different forms as described herein may be used as neoadjuvant (prior), adjunctive (during), and/or adjuvant (after) therapy with surgery, radiation (brachytherapy or external beam), or other therapies (*e*.*g*., HIFU), and in combination with chemotherapies, androgen ablation, antiandrogens or any other therapeutic approach.

With respect to combination therapies, one embodiment of the present disclosure provides a combination of any one or more of a compound of Formula I with one or more currently-used or experimental pharmacological therapies which are or may be utilized to treat any of the above disease states (e.g., castration-resistant prostate cancer or Kennedy's disease). Methods, uses and pharmaceutical compositions comprising the above combination are also provided.

Surprisingly, it has been found that the disclosed compounds, which interfere with the AR principally through binding to the N-terminus of the AR, demonstrate beneficial synergistic therapeutic effects when used in concert with existing approved and in-development agents. That is, the biological impact of using the agents in concert with one another produces a biological and therapeutic effect which is greater than the simple additive effect of each of them separately.

Accordingly, one embodiment comprises the use of the disclosed compounds in combination therapy with one or more currently-used or experimental pharmacological therapies which are utilized for treating the above disease states irrespective of the biological mechanism of action of such pharmacological therapies, including without limitation pharmacological therapies which directly or indirectly inhibit the androgen receptor, pharmacological therapies which are cyto-toxic in nature, and pharmacological therapies which interfere with the biological production or function of androgen (hereinafter, the "Other Therapeutic Agents"). By "combination therapy" is meant the administration of any one or more of a compound of Formula I with one or more of another therapeutic agent to the same patient such that their pharmacological effects are contemporaneous with one another, or if not contemporaneous, that their effects are synergistic with one another even though dosed sequentially rather than contemporaneously.

Such administration includes without limitation dosing of one or more of a compound of Formula I and and one or more of the Other Therapeutic Agent(s) as separate agents without any comingling prior to dosing, as well as formulations which include one or more Other Androgen-Blocking Therapeutic Agents mixed with one or more compound of Formula I as a pre-mixed formulation. Administration of the compound(s) of Formula I in combination with Other Therapeutic Agents for treatment of the above disease states also includes dosing by any dosing method including without limitation, intravenous delivery, oral delivery, intra-peritoneal delivery, intra-muscular delivery, or intra-tumoral delivery.

In another aspect of the present disclosure, the one or more of the Other Therapeutic Agent may be administered to the patient before administration of the compound(s) of Formula I. In another embodiment, the compound(s) of Formula I may be co-administered with one or more of the Other Therapeutic Agents. In yet another aspect, the one or more Other Therapeutic Agent may be administered to the patient after administration of the compound(s) of Formula I.

It is fully within the scope of the disclosure that the ratio of the doses of compound(s) of Formula I to that of the one or more Other Therapeutic Agents may or may not equal to one and may be varied accordingly to achieve the optimal therapeutic benefit.

For greater clarity the compound(s) of Formula I that are combined with the one or more Other Therapeutic Agents for improved treatment of the above disease states may comprise, but are not limited to any compound having a structure of Formula I, including those compounds shown in Table 2.

The Other Therapeutic Agents include without limitation any pharmacological agent which is currently approved by the FDA in the U.S. (or elsewhere by any other regulatory body) for use as pharmacological treatment of any of the above disease states, or which is currently being used experimentally as part of a clinical trial program that relates to the above disease states. Non-limiting examples of the Other Pharmacological Agents comprise, without limitation: the chemical entity known as **MDV3100** (4-(3-(4-cyano-3-(trifluoromethyl)phenyl)-5,5-dimethyl-4-oxo-2-thioxoimidazolidin-1-yl)-2-fluoro-N-methylbenzamide) and related compounds, which appears to be a blocker of the AR LBD and is currently in development as a treatment for prostate cancer; the chemical entity known as Orteronel **(TAK-700)** and related compounds which appears to block androgen synthesis, the chemical entity known as **TOK 001** (Galeterone) and related compounds which appears to be a blocker of the AR LBD, and a CYP17 lyase inhibitor, and also appears to decrease overall androgen receptor levels in prostate cancer cells. **TOK 001** is currently in development as a treatment for prostate cancer; the chemical entity known as **ARN-509** and related compounds which appears to be a blocker of the AR LBD and is currently in development as a treatment for prostate cancer; the chemical entity known as **abiraterone acetate** (or CB-7630; (3S,8R,9S,10R,13S,14S)-10,13-dimethyl-17-(pyridin-3-yl) 2,3,4,7,8,9,10,11,12,13,14,15-dodecahydro-1H-cyclopenta[a]phenanthren-3-ol), and related molecules, which appears to block the production of androgen and is currently in development for the treatment of prostate cancer; the chemical entity known as **bicalutamide** (N-[4-cyano-3-(trifluoromethyl)phenyl]-3-[(4-fluorophenyl)sulfonyl]-2-hydroxy-2-methylpropanamide) and related compounds, which appears to be a blocker of the AR LBD and which is currently used to treat prostate cancer, the chemical entity known as **nilutamide** (5,5-dimethyl-3-[4-nitro-3-(trifluoromethyl)phenyl] imidazolidine-2,4-dione) and related compounds, which appears to be a blocker of the AR LBD and which is currently used to treat prostate cancer, the chemical entity known as **flutamide** (2-methyl-N-[4-nitro-3-(trifluoromethyl)phenyl]-propanamide) and related compounds, which appears to be a blocker of the AR LBD and which is currently used to treat prostate cancer, the chemical entities know as **cyproterone acetate** (6-chloro-1β,2β-dihydro-17-hydroxy-3'H-cyclopropa[1,2]pregna-4,6-diene-3,20-dione) and related compounds, which appears to be a blocker of the AR LBD and which is currently used to treat prostate cancer, the chemical entity known as **docetaxel** (Taxotere; 1,7β,10β-trihydroxy-9-oxo-5β,20-epoxytax-11-ene-2a,4,13a-triyl 4-acetate 2-benzoate 13-{(2R,3S)-3-[(tert-butoxycarbonyl)amino]-2-hydroxy-3-phenylpropanoate}) and related compounds, which appears to be a cytotoxic antimicrotubule agent and is currently used in combination with prednisone to treat prostate cancer, the chemical entity known as **Bevacizumab** (Avastin), a monoclonal antibody that recognizes and blocks vascular endothelial growth factor A (VEGF-A) and may be used to treat prostate cancer, the chemical entity known as **OSU-HDAC42** ((S)-(+)-N-hydroxy-4-(3-methyl-2-phenylbutyrylamino)-benzamide), and related compounds, which appears to act as a histone deacetylase inhibitor, and is currently being developed as a treatment for prostate cancer, the chemical entity known as **VITAXIN** which appears to be a monoclonal antibody against the vascular integrin ανβ3 to prevent angiogenesis, and which may be used to treat prostate cancer, the chemical entity known as **sunitumib** (N-(2-diethylaminoethyl)-5-[(Z)-(5-fluoro-2-oxo-1H-indol-3-ylidene)methyl]-2,4-dimethyl-1H-pyrrole-3-carboxamide) and related compounds, which appears to inhibit multiple receptor tyrosine kinases (RTKs) and may be used for treatment of prostate cancer, the chemical entity known as **ZD-4054** (N-(3-Methoxy-5-methylpyrazin-2-yl)-2-[4-(1,3,4-oxadiazol-2-yl)phenyl]pyridin-3-sulfonamid) and related compounds, which appears to block the edta receptor and which may be used for treatment of prostate cancer, the chemical entity known as **Cabazitaxel (XRP-6258),** and related compounds, which appears to be a cytotoxic microtubule inhibitor, and which is currently used to treat prostate cancer; the chemical entity known as **MDX-010 (Ipilimumab),** a fully human monoclonal antibody that binds to and blocks the activity of CTLA-4 which is currently in development as an immunotherapeutic agent for treatment of prostate cancer; the chemical entity known as **OGX 427** which appears to target HSP27 as an antisense agent, and which is currently in development for treatment of prostate cancer; the chemical entity known as **OGX 011** which appears to target clusterin as an antisense agent, and which is currently in development as a treatment for prostate cancer; the chemical entity known as **finasteride** (Proscar, Propecia; N-(1,1-dimethylethyl)-3-oxo-(5α,17β)-4-azaandrost-1-ene-17-carboxamide), and related compounds, which appears to be a 5-alpha reductase inhibitor that reduces levels of dihydrotestosterone, and may be used to treat prostate cancer; the chemical entity known as **dutasteride** (Avodart; 5α, 17β)-N-{2,5 bis(trifluoromethyl) phenyl}-3-oxo-4-azaandrost-1-ene-17-carboxamide) and related molecules, which appears to be a 5-alpha reductase inhibitor that reduces levels of dihydrotestosterone, and may be used in the treatment of prostate cancer; the chemical entity known as **turosteride** ((4aR,4bS,6aS,7S,9aS,9bS,11aR)-1,4a,6a-trimethyl-2-oxo-N-(propan-2-yl)-N-(propan-2 ylcarbamoyl)hexadecahydro-1H-indeno[5,4-f]quinoline-7-carboxamide), and related molecules, which appears to be a 5-alpha reductase inhibitor that reduces levels of dihydrotestosterone and may be used in the treatment of prostate cancer; the chemical entity known as **bexlosteride** (LY-191,704; (4aS,10bR)-8-chloro-4-methyl-1,2,4a,5,6,10b-hexahydrobenzo[f]quinolin-3-one), and related compounds, which appears to be a 5-alpha reductase inhibitor that reduces levels of dihydrotestosterone and may be used in the treatment of prostate cancer; the chemical entity known as **izonsteride** (LY-320,236; (4aR,10bR)-8-[(4-ethyl-1,3-benzothiazol-2-yl)sulfanyl]-4,10b-dimethyl-1,4,4a,5,6,10b-hexahydrobenzo[f]quinolin-3(2H)-one) and related compounds, which appears to be a 5-alpha reductase inhibitor that reduces levels of dihydrotestosterone and may be used for the treatment of prostate cancer; the chemical entity known as **FCE 28260** and related compounds, which appears to be a 5-alpha reductase inhibitor that reduces levels of dihydrotestosterone and may be used for the treatment of prostate cancer; the chemical entity known as **SKF105,111,** and related compounds, which appears to be a 5-alpha reductase inhibitor that reduces levels of dihydrotestosterone and may be used for treatment of prostate cancer.

In general, compounds of the invention should be used without causing substantial toxicity. Toxicity of the compounds of the invention can be determined using standard techniques, for example, by testing in cell cultures or experimental animals and determining the therapeutic index, *i.e.,* the ratio between the LD50 (the dose lethal to 50% of the population) and the LD100 (the dose lethal to 100% of the population). In some circumstances however, such as in severe disease conditions, it may be necessary to administer substantial excesses of the compositions. Some compounds of this invention may be toxic at some concentrations. Titration studies may be used to determine toxic and non-toxic concentrations. Toxicity may be evaluated by examining a particular compound's or composition's specificity across cell lines using PC3 cells as a negative control that do not express functional AR. Animal studies may be used to provide an indication if the compound has any effects on other tissues. Systemic therapy that targets the AR will not likely cause major problems to other tissues since antiandrogens and androgen insensitivity syndrome are not fatal.

Compounds as described herein may be administered to a subject. As used herein, a "subject" may be a human, non-human primate, mammal, rat, mouse, cow, horse, pig, sheep, goat, dog, cat and the like. The subject may be suspected of having or at risk for having a cancer, such as prostate cancer, breast cancer, ovarian cancer, salivary gland carcinoma, or endometrial cancer, or suspected of having or at risk for having acne, hirsutism, alopecia, benign prostatic hyperplasia, ovarian cysts, polycystic ovary disease, precocious puberty, spinal and bulbar muscular atrophy, or age-related macular degeneration. Diagnostic methods for various cancers, such as prostate cancer, breast cancer, ovarian cancer, salivary gland carcinoma, or endometrial cancer, and diagnostic methods for acne, hirsutism, alopecia, benign prostatic hyperplasia, ovarian cysts, polycystic ovary disease, precocious puberty, spinal and bulbar muscular atrophy, or age-related macular degeneration and the clinical delineation of cancer, such as prostate cancer, breast cancer, ovarian cancer, salivary gland carcinoma, or endometrial cancer, diagnoses and the clinical delineation of acne, hirsutism, alopecia, benign prostatic hyperplasia, ovarian cysts, polycystic ovary disease, precocious puberty, spinal and bulbar muscular atrophy, or age-related macular degeneration are known to those of ordinary skill in the art.

Compounds described herein may be used for treatment of at least one indication selected from the group consisting of: prostate cancer, breast cancer, ovarian cancer, endometrial cancer, salivary gland carcinoma, hair loss, acne, hirsutism, ovarian cysts, polycystic ovary disease, precocious puberty, spinal and bulbar muscular atrophy, and age-related macular degeneration. Compounds described herein may be used for treatment of prostate cancer. Compounds described herein may be used for treatment of castration-resistant prostate cancer. Compounds described herein may be used for treatment of androgen-dependent prostate cancer. Compounds described herein may be used for preparation of a medicament for treatment of at least one indication selected from the group consisting of: prostate cancer, breast cancer, ovarian cancer, endometrial cancer, salivary gland carcinoma, hair loss, acne, hirsutism, ovarian cysts, polycystic ovary disease, precocious puberty, spinal and bulbar muscular atrophy, and age-related macular degeneration. Compounds described herein may be used for the preparation of a medicament for treatment of prostate cancer. Compounds described herein may be used for the preparation of a medicament for treatment of castration-resistant prostate cancer. Compounds described herein may be used for the preparation of a medicament for treatment of androgen-dependent prostate cancer. Compounds described herein may be used in a method for treatment of at least one indication selected from the group consisting of: prostate cancer, breast cancer, ovarian cancer, endometrial cancer, salivary gland carcinoma, hair loss, acne, hirsutism, ovarian cysts, polycystic ovary disease, precocious puberty, spinal and bulbar muscular atrophy, and age-related macular degeneration. The method may comprise administering to a subject in need thereof an effective amount of a compound described herein. Compounds described herein may be used in a method of treatment of prostate cancer, the method comprising administering to a subject in need thereof an effective amount of a compound described herein. Compounds described herein may be used in a method of treatment of castration resistant prostate cancer, the method comprising administering to a subject in need thereof an effective amount of a compound described herein. Compounds described herein may be used in a method of treatment of androgen-dependent prostate cancer, the method comprising administering to a subject in need thereof an effective amount of a compound described herein.

Compounds described herein may also be used in assays and for research purposes. Definitions used include ligand-dependent activation of the androgen receptor (AR) by androgens such as dihydrotestosterone (DHT) or the synthetic androgen (R1881) used for research purposes. Ligand-independent activation of the AR refers to transactivation of the AR in the absence of androgen (ligand) by, for example, stimulation of the cAMP-dependent protein kinase (PKA) pathway with forskolin (FSK). Some compounds and compositions of this invention may inhibit both FSK and androgen (*e*.*g*., R1881) induction of ARE-luciferase (ARE-luc). Constituative activity of the AR refers to splice variants lacking the AR ligand-binding domain. Such compounds may block a mechanism that is common to both ligand-dependent and ligand-independent activation of the AR, as well as constitutively active splice variants of the AR that lack ligand-binding domain. This could involve any step in activation of the AR including dissociation of heatshock proteins, essential posttranslational modifications (*e*.*g*., acetylation, phosphorylation), nuclear translocation, protein-protein interactions, formation of the transcriptional complex, release of co-repressors, and/or increased degradation. Some compounds and compositions of this invention may inhibit ligand-only activity and may interfere with a mechanism specific to ligand-dependent activation (*e*.*g*., accessibility of the ligand binding domain (LBD) to androgen). Numerous disorders in addition to prostate cancer involve the androgen axis (*e*.*g*., acne, hirsutism, alopecia, benign prostatic hyperplasia) and compounds interfering with this mechanism may be used to treat such conditions. Some compounds and compositions of this invention may only inhibit FSK induction and may be specific inhibitors to ligand-independent activation of the AR. These compounds and compositions may interfere with the cascade of events that normally occur with FSK and/or PKA activity or any downstream effects that may play a role on the AR (*e*.*g*., FSK increases MAPK activity which has a potent effect on AR activity). Examples may include an inhibitor of cAMP and or PKA or other kinases. Some compounds and compositions of this invention may induce basal levels of activity of the AR (no androgen or stimulation of the PKA pathway). Some compounds and compositions of this invention may increase induction by R1881 or FSK. Such compounds and compositions may stimulate transcription or transactivation of the AR. Some compounds and compositions of this invention may inhibit activity of the androgen receptor. Interleukin-6 (IL-6) also causes ligand-independent activation of the AR in LNCaP cells and can be used in addition to FSK.

Compounds for use in the present invention may be obtained from medical sources or modified using known methodologies from naturally occurring compounds. In addition, methods of preparing or synthesizing compounds of the present invention will be understood by a person of skill in the art having reference to known chemical synthesis principles. For example, Auzou et al 1974 European Journal of Medicinal Chemistry 9(5), 548-554 describes suitable synthetic procedures that may be considered and suitably adapted for preparing compounds of any one of the Formula I to XXI as set out above. Other references that may be helpful include: Debasish Das, Jyh-Fu Lee and Soofin Cheng "Sulfonic acid functionalized mesoporous MCM-41 silica as a convenient catalyst for Bisphenol-A synthesis" Chemical Communications, (2001) 2178-2179; US Patent 2571217 Davis, Orris L.; Knight, Horace S.; Skinner, John R. (Shell Development Co.) "Halohydrin ethers of phenols." (1951); and Rokicki, G.; Pawlicki, J.; Kuran, W. "Reactions of 4-chloromethyl-1,3-dioxolan-2-one with phenols as a new route to polyols and cyclic carbonates." Journal fuer Praktische Chemie (Leipzig) (1985) 327, 718-722.

For example, compounds of the present invention may be prepared with reference to the following General Reaction Scheme I:

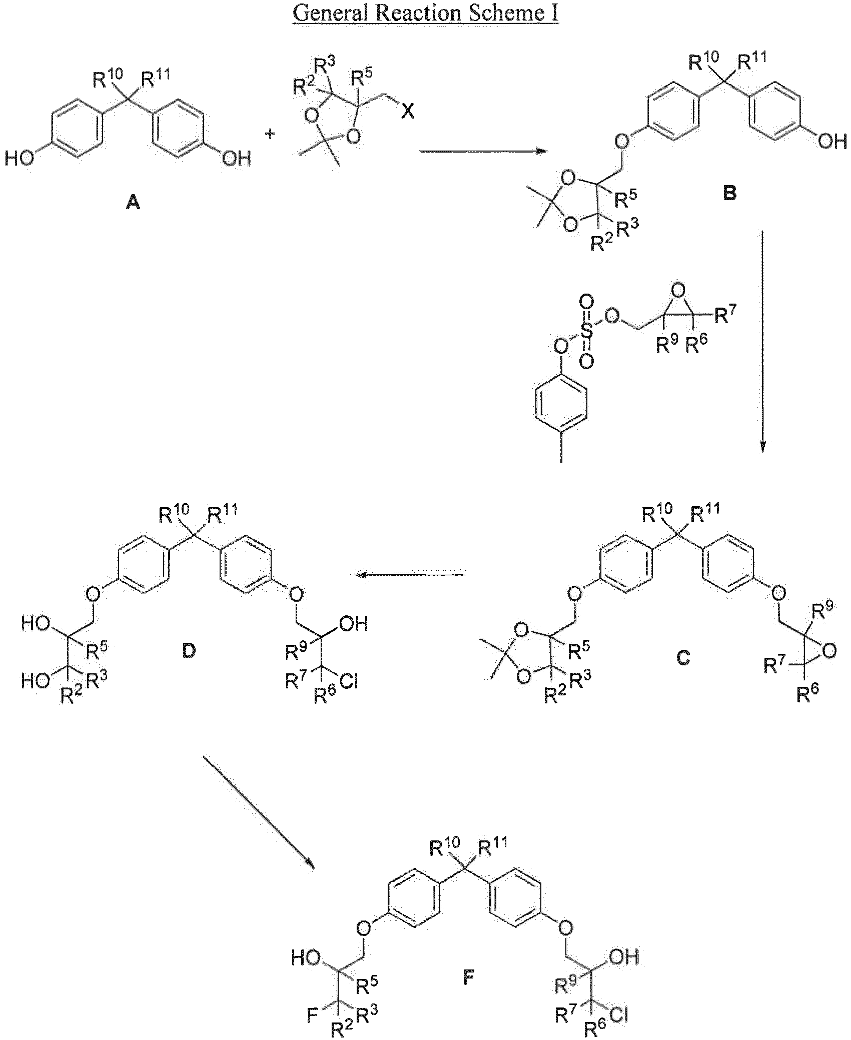

Referring to Synthetic Scheme 1, compounds of structure A can be purchased from commercial sources or prepared according to methods known in the art. Reaction of A with an appropriately substituted 1,3-dioxolane, wherein X is an appropriate leaving group (*e.g*., chloro), yields compounds of structure B. Optically pure or racemic dioxolanes may be employed to yield the desired stereochemistry. Epoxidation of B with an appropriate reagent, for example an appropriately substituted glycidyl tosylate, results in compounds of structure C. Various epoxidation reagents may be employed, including optically pure reagents which yield optically pure epoxides (e.g., + or - glycidyl tosylate). Treatment of C with an appropriate ring-opening reagent, for example CeCl₃x7H₂O, yields D.

Fluorination of D results in F. Methods for such fluorination are well known. For example, in one embodiment a fluorine atom is introduced by treatment with diethylaminosulfurtrifluoride (DAST) or Xtalfluor-E or M (see J. Org. Chem. 2010, 75, 3401-3411). In other embodiments, the hydroxyl moiety in **D** may be converted to an appropriate leaving group, for example by reaction with tosyl chloride or mesyl anhydride, followed by reaction with [K⁺/2,2,2-cryptand]F⁻ or tetrabutylammonium fluoride. Other methods for fluorination of D are known to those of skill in the art. For descriptions of fluorination procedures see J. Org. Chem. 2010, 75, 3401-3411, Bioorg. Med. Chem. 2009, 17, 7441-7448, and J. Med. Chem. 1990, 33, 2430-2437.

One skilled in the art will recognize that variations to the order of the steps and reagents discussed in reference to Synthetic Scheme I are possible. For example, epoxidation may precede dioxalone formation. Further, fluorine atoms may be introduced via any number of reagents, and fluorination is not limited to those methods depicted or described above. Methods for such fluorination are well known in the art. Finally, prodrugs of Formula I can be prepared by functionalizing a free hydroxyl in structure D or F. Methods for such functionalization are well-known in the art, for example reaction with an acid chloride analogue of a moiety from Table 1 or any other suitable reagent. Methodologies for preparation of compounds of Formula I are described in more detail in the following non-limiting exemplary schemes.

Various alternative embodiments and examples of the invention are described herein. These embodiments and examples are illustrative and should not be construed as limiting the scope of the invention.

### EXAMPLES

Examples not pertaining to the invention are for illustrative purposes only.

All non-aqueous reactions were performed in flame-dried round bottomed flasks. The flasks were fitted with rubber septa and reactions were conducted under a positive pressure of argon unless otherwise specified. Stainless steel syringes were used to transfer air- and moisture-sensitive liquids. Flash column chromatography was performed as described by Still et al. (Still, W. C.; Kahn, M.; Mitra, A. J. Org. Chem. 1978, 43, 2923) using 230-400 mesh silica gel. Thin-layer chromatography was performed using aluminum plates pre-coated with 0.25 mm 230-400 mesh silica gel impregnated with a fluorescent indicator (254 nm). Thin-layer chromatography plates were visualized by exposure to ultraviolet light and a "Seebach" staining solution (700 mL water, 10.5 g Cerium (IV) sulphate tetrahydrate, 15.0 g molybdato phosphoric acid, 17.5 g sulphuric acid) followed by heating (~1 min) with a heating gun (~250 °C). Organic solutions were concentrated on Büchi R-114 rotatory evaporators at reduced pressure (15-30 torr, house vacuum) at 25-40 °C.

Commercial regents and solvents were used as received. All solvents used for extraction and chromatography were HPLC grade. Normal-phase Si gel Sep paks^{™} were purchased from waters, Inc. Thin-layer chromatography plates were Kieselgel 60F₂₅₄. All synthetic reagents were purchased from Sigma Aldrich and Fisher Scientific Canada.

Proton nuclear magnetic resonance (¹H NMR) spectra were recorded at 25 °C using a Bruker 400 with inverse probe and Bruker 400 spectrometers, are reported in parts per million on the δ scale, and are referenced from the residual protium in the NMR solvent (DMSO-d₆: δ 2.50 (DMSO-d₅), CDCl₃: δ 7.24 (CHCl₃)). Carbon-13 nuclear magnetic resonance (¹³C NMR) spectra were recorded with a Bruker 400 spectrometer, are reported in parts per million on the δ scale, and are referenced from the carbon resonances of the solvent (DMSO-*d*₆: δ 39.51, CDCl₃: δ 77.00). Spectral features are tabulated in the following order: chemical shift (δ, ppm); multiplicity (s = singlet, d = doublet, t = triplet, m = multiplet, br = broad); coupling constant (*J,* Hz, number of protons).

LNCaP cells were employed initially for all experiments because they are well-differentiated human prostate cancer cells in which ligand-independent activation of the AR by FSK has been characterized (Nazareth et al 1996 J. Biol. Chem. 271, 19900-19907; and Sadar 1999 J. Biol. Chem. 274, 7777-7783). LNCaP cells express endogenous AR and secrete prostate-specific antigen (PSA) (Horoszewicz et al 1983 Cancer Res. 43, 1809-1818). LNCaP cells can be grown either as monolayers in cell culture or as tumors in the well-characterized xenograft model that progresses to castration resistance in castrated hosts (Sato et al 1996 J. Steroid Biochem. Mol. Biol. 58, 139-146; Gleave et al 1991 Cancer Res. 51, 3753-3761; Sato et al 1997 Cancer Res. 57, 1584-1589; and Sadar et al 2002 Mol. Cancer Ther. 1(8), 629-637). R1881 was employed since it is stable and avoids problems associated with the labile physiological ligand dihydrotestosterone (DHT). Reporter specificity may be determined using several alternative reporter gene constructs. Some well characterized ARE-driven reporter gene constructs that have been used extensively are the PSA (6.1 kb) enhance/promoter which contains several AREs and is highly inducible by androgens as well as by FSK (Ueda et al 2002 A J. Biol. Chem. 277, 7076-7085) and the ARR3-thymidine kinase (tk)-luciferase, which is an artificial reporter construct that contains three tandem repeats of the rat probasin ARE1 and ARE2 regions upstream of a luciferase reporter (Snoek et al 1996 J. Steroid Biochem. Mol. Biol. 59, 243-250).

### EXAMPLE 1

### SYNTHESIS OF (S)-4-(2-(4-((2,2-DIMETHYL-1,3-DIOXOLAN-4-YL)METHOXY)PHENYL)PROPAN-2-YL)PHENOL (A)

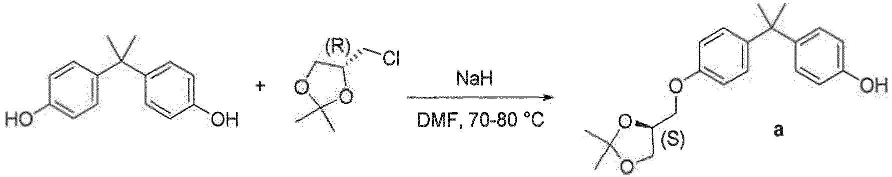

Sodium hydride (60% dispersion in mineral oil, 1750 mg, 43.80 mmol, 1.0 equiv) was added slowly to a stirred solution of Bisphenol A (10000 mg, 43.80 mmol, 1 equiv) in anhydrous dimethyl formamide (30 mL), at room temperature, and the contents were stirred under an atmosphere of argon for 20 min. (*R*)-(+)-4-chloromethyl-2,2-dimethyl-1,3-dioxolane 98% (7.10 mL, 52.56 mmol, 1.2 equiv) was added via syringe and the mixture was allowed to react at 70-80 °C for 40 h. *Caution! Using sodium hydride in warmed N,N dimethylformamide could result in deflagration and fire.* Next, the reaction was quenched by the addition of a saturated solution of ammonium chloride (10 mL), and the mixture was extracted with ethyl acetate (3 x 20 mL). The organic layer was washed with deionized water (25 mL), dried over anhydrous magnesium sulfate, filtered and concentrated under reduced pressure. The resulting residue was purified by flash column chromatography on silica gel (eluent: 10% ethyl acetate in hexane) to provide compound (**a**) (2*S*) (3560 mg, 24%, 25-30% conversion) as a foam. Other stereoisomers (including racemic isomers) can be prepared by starting with the appropriate dioxolane reagent. ¹H NMR (400 MHz, CDCl₃): δ 7.15-7.13 (m, 2H), 7.08-7.06 (m, 2H), 6.82-6.80 (m, 2H), 6.75-6.74 (m, 2H), 4.50-4.45 (m, 1H), 4.19-4.15 (dd, *J* = 8.4, 6.4, 1H), 4.07-4.03 (dd, *J=* 9.6, 5.6, 1H), 3.94-3.88 (m, 2H), 1.63 (s, 6H), 1.47 (s, 3H), 1.41 (s, 3H); ¹³C NMR (100 MHz, CDCl₃):δ 156.5, 153.9, 143.9, 142.9, 128.0, 127.9, 114.9, 114.9, 114.0, 109.9, 74.2, 68.9, 67.1, 41.8, 31.2, 26.9, 25.5; HRMS (ESI) (*m*/*z*):calc'd for C₂₁H₂₇O₄ [M+H]⁺: 343.1909, found: 343.1904.

### EXAMPLE 2

### SYNTHESIS OF (S)-2,2-DIMETHYL-4-((4-(2-(4-((R)-OXIRAN-2-YLMETHOXY)PHENYL)PROPAN-2-YL)PHENOXY)METHYL)-1,3-DIOXOLANE (B)

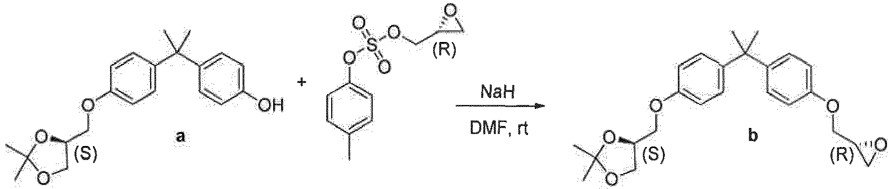

Sodium hydride (60% dispersion in mineral oil, 391 mg, 9.78 mmol, 1.5 equiv) was added slowly to a stirred solution of derivative (**a**) (2230 mg, 6.52 mmol, 1 equiv) in anhydrous dimethyl formamide (15 mL), at room temperature, and the contents were stirred under an atmosphere of argon for 30 min. A solution of (2*R*)-(-)-glycidyl tosylate 98% (2230 mg, 9.78 mmol, 1.5 equiv) in anhydrous dimethyl formamide (5 mL) was added via syringe, and the mixture was allowed to react at room temperature for 16 h. The reaction was then quenched by addition of a saturated solution of ammonium chloride (10 mL), and the mixture was extracted with ethyl acetate (3 x 20 mL). The organic layer was washed with deionized water (20 mL), dried over anhydrous magnesium sulfate, filtered and concentrated under reduced pressure. The resulting residue was purified by flash column chromatography on silica gel (eluent: 20% to 40% ethyl acetate in hexane) to provide (**b**) (2*S*,20*R*) (2.53 g, 94%) as a clear foam. Other diastereomers or racemic mixtures are prepared by using and appropriate tosylate. ¹HNMR (400 MHz, DMSO-*d*₆): δ 7.11-7.08 (dd, *J=* 8.8, 1.2, 4H), 6.85-6.82 (dd, *J=* 6.4, 3.2, 4H), 4.41-4.35 (m, 1H), 4.28-4.25 (dd, *J* = 11.2, 2.4, 1H), 4.09-4.06 (dd, *J* = 8.4, 6.8, 1H), 3.95-3.94 (m, 2H), 3.81-3.72 (m, 2H), 3.32-3.29 (m, 1H), 2.82 (t, *J* = 4.8, 1H), 2.69-2.67 (dd, *J* = 4.8, 2.4, 1H), 1.57 (s, 6H), 1.35 (s, 3H), 1.29 (s, 3H); ¹³C NMR (100 MHz, DMSO-*d*₆): δ 156.1, 156.0, 142.9, 142.8, 127.4, 113.9, 113.8, 108.8, 73.7, 68.8, 68.6, 65.8, 49.7, 43.7, 41.2, 30.7, 26.6, 25.4; HRMS (ESI) (*m*/*z*): calc'd for C₂₄H₃₀O₅Na [M+Na]⁺: 421.1991, found: 421.1986.

### EXAMPLE 3

### SYNTHESIS OF (R)-3-(4-(2-(4-((S)-4-CHLORO-3-HYDROXYBUTYL)PHENYL)PROPAN-2-YL)PHENOXY)PROPANE-1,2-DIOL (C)

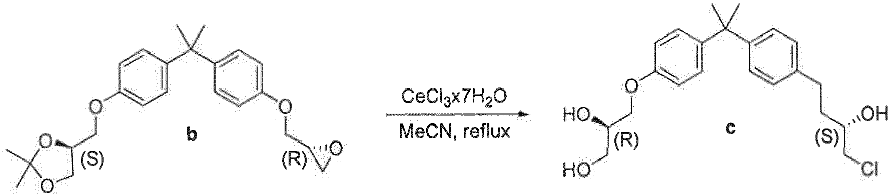

To a solution of derivative (**b**) (2530 mg, 6.34 mmol, 1 equiv) in acetonitrile (25 mL) was added CeCl₃·7H₂O (5910 mg, 15.87 mmol, 2.5 equiv), and the mixture was refluxed for 20 h. The resulting white paste was filtered and washed with ethyl acetate, and the clear suspension was concentrated under reduced pressure. The resulting residue was purified by flash column chromatography on silica gel (eluent: 20% hexane in ethyl acetate to 100% ethylacetate) and Si gel Sep pak (10g, eluent: 50% hexane in ethyl acetate to 80% ethylacetate) to provide (c) (2*R*,20*S*) (2250 mg, 90%) as a transparent foam. ¹H NMR (400 MHz, DMSO-*d*₆): δ 7.09 (dd, *J* = 8.8, 4.0, 4H), 6.82 (t, *J=* 8.4, 4H), 5.52 (d, *J* = 5.2, 1H), 4.90 (d, *J=* 4.8, 1H), 4.63 (t, *J* = 5.6, 1H), 4.03-3.98 (m, 1H), 3.96-3.92 (m, 3H), 3.82-3.72 (m, 3H), 3.67-3.63 (dd, *J=* 11.2, 5.6, 1H), 3.42 (t, *J* = 6.0, 2H), 1.57 (s, 6H); ¹³C NMR (100 MHz, DMSO-*d*₆):δ 156.5, 156.1, 142.9, 142.4, 127.4, 127.4, 113.9, 113.8, 69.9, 69.4, 68.8, 68.6, 62.7, 46.8, 41.1, 30.7; HRMS (ESI) *(m*/*z):* calc'd for C₂₁H₂₇O₅NaCl [M+Na]⁺: 417.1445, found: 417.1440.

### EXAMPLE 4

### SYNTHESIS OF (S)-1-CHLORO-4-(4-(2-(4-((S)-3-FLUORO-2-HYDROXYPROPOXY)PHENYL)PROPAN-2-YL)PHENYL)BUTAN-2-OL (1C)

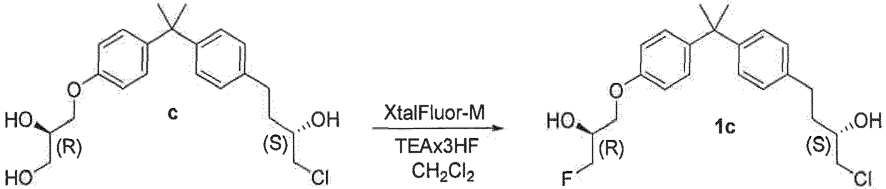

To a solution of (c**)** (100 mg, 0.25 mmol) in dichloromethane (2.0 mL) were successively added triethylamine trihydrofluoride (84 µL, 0.51 mmol) and XTalFluor-M (123 mg, 0.51 mmol). After 3 h, the reaction mixture was quenched at room temperature with a 5% aqueous sodium bicarbonate solution and stirred for 15 min, and the resulting mixture was extracted twice with dichloromethane. The organic phases were combined, dried over anhydrous magnesium sulfate, and filtered. Solvents were evaporated, and the resulting crude material was purified by silica gel flash chromatography (eluent: 0 to 5% ethyl acetate in dichloromethane) to provide (**1c**) (17 mg, 17%) as pale foam. ¹H NMR (400 MHz, DMSO-*d*₆): δ 7.09 (d, *J* = 8.4, 4H), 6.83 (d, *J* = 8.4, 4H), 5.52 (d, *J* = 5.2, 1H), 5.41 (d, *J* = 5.2, 1H), 4.56-4.48 (ddd, *J* = 18.8, 9.2, 3.6, 1H), 4.45-4.36 (ddd, *J* = 19.2, 10.0, 4.0, 1H), 4.05-3.97 (m, 2H), 3.93-3.90 (m, 4H), 3.75-3.72 (dd, *J* = 10.8, 4.0, 1H), 3.67-3.63 (dd, *J* = 11.2,5.2, 1H), 1.57 (s, 6H); ¹⁹F NMR (282 MHz, CDCl₃):δ -232.8-233.2 (td, *J* = -47.9, -19.7, 1F); ¹⁹F NMR ¹H decoupling (282 MHz, CDCl₃):δ -233.0 (s, 1F); ¹³C NMR (100 MHz, DMSO-*d*₆): δ 156.1, 142.8, 127.4, 113.9, 85.3, 83.6 (d, *J=* 166.0), 68.9, 68.6, 68.0, 67.9 (d, *J* = 8.0), 67.7, 67.5 (d, *J* = 20.0), 46.8, 41.2, 30.7.

Different stereoisomers were prepared according to the above procedures (*i.e*., Examples 1-4) by employing the appropriate chiral reagent.

### EXAMPLE 5

### INHIBITION OF ANDROGEN-DEPENDENT PROLIFERATION

As noted in Example 9, comparative compound (Ac) is the most potent and least toxic of the comparative compounds (*i.e*., compounds lacking the chloro and fluoro substituents of Formula (I)). Accordingly, a series of experiments was designed to evaluate the potency and toxicity of various compounds of Formula (I) relative to comparative compound (Ac). To this end, androgen-dependent proliferation of LNCaP cells treated with 0.1 nM R1881 for 3 days compared to PC3 cell viability at 2 days was determined with increasing concentrations of comparative compound (Ac) or compound 1 using the AlamarBlue Proliferation Assay. LNCaP human prostate cancer cells were maintained in phenol red-free RPMI 1640 medium with 0.5% (v/v) fetal bovine serum, while PC3 human prostate cancer cells were cultured in phenol red DMEM medium with 0.5% (v/v) fetal bovine serum at 37 °C. Cells were seeded in 96-well plates for 24 hrs before pre-treatment for 1 hr with comparative compound (Ac) or compound 1 followed by addition of 0.1 nM R1881 (synthetic androgen) for LNCaP cells. LNCaP cells were incubated for 72 hours with R1881, while the duration of the experiment was 48 hours for PC3 cells. AlamarBlue reagent was added to the cells prior to incubation and measuring fluorescence at 570 nm.

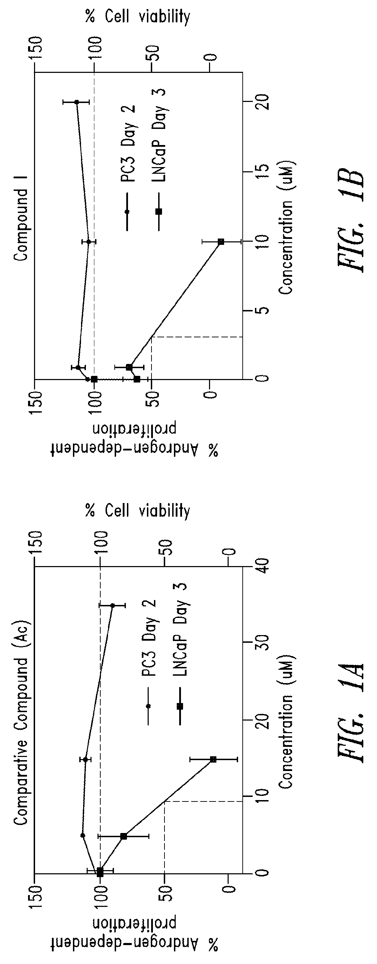

Figures 1A and 1B present data from the above experiment. Inspection of Figures 1A and 1B shows that compound 1 inhibits androgen-dependent proliferation of LNCaP cells at lower concentrations (*i.e*., more potent inhibitor) compared to comparative compound (Ac).

### EXAMPLE 6

### INHIBITION OF ANDROGEN RECEPTOR N-TERMINAL DOMAIN TRANSACTIVATION

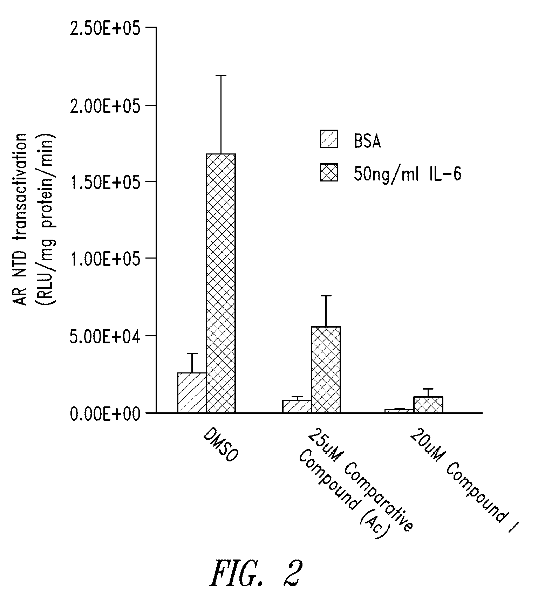

Inhibition of androgen receptor N-terminal domain transactivation was determined as follows. LNCaP cells were cotransfected with 5xGA14UAS-TATA-luciferase and AR (1-558)-Gal4DBD prior to preincubation with indicated concentrations of comparative compound (Ac) (25 µm) or EPI-0011 compound 1 (20 µm) and then treated with 50ng/ml interleukin-6 (IL-6) or BSA (bovine serum albumin - control). The data in Figure 2 demonstrate the more potent inhibition activity of compound 1 relative to comparative compound (Ac).

### EXAMPLE 7

### INHIBITION OF TUMOR GROWTH

Growth inhibition of VCaP xenografts in castrated mice was determined as follow. Male NOD-SCID mice bearing subcutaneous VCaP tumors were castrated when tumor volume was ~ 100mm³. Seven days after castration, the hosts were injected intravenously with 50mg/kg body weight of comparative compound (Ac), compound 1, compound 1c or compound 1d every other day for a total of seven doses. The experiment ended two days after the last dose. Change in tumor volume was determined as the change at day 14 compared to the day of 1^{st} injection. DMSO was used as the vehicle control.

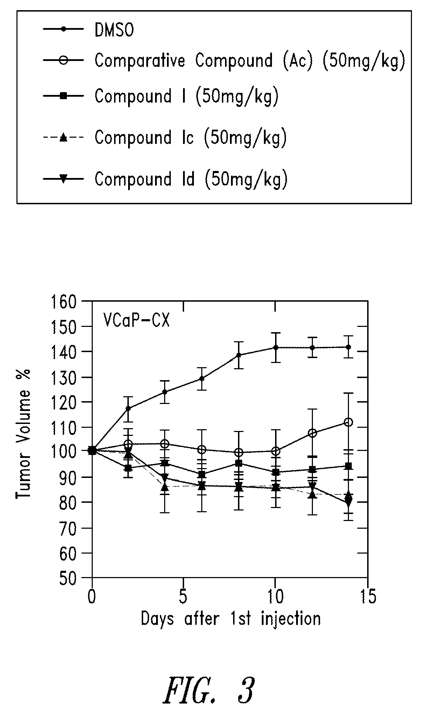

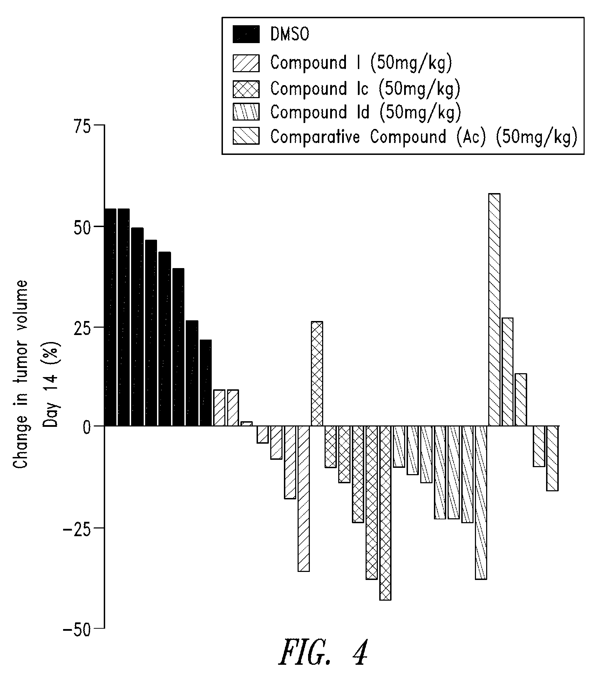

Growth Curves of VCaP xenografts in castrated mice determined according to the above experiments are shown in Figure 3. Figure 4 depicts the change in tumor volume at day 14. Mice treated with compounds 1, 1c and 1d all experienced an increased loss in tumor volume compared to mice treated with comparative compound (Ac).

### EXAMPLE 8

### CHANGE IN BODY WEIGHT

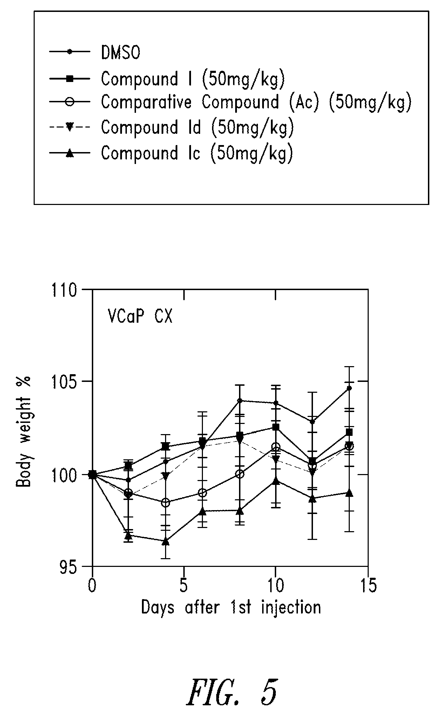

Compounds effects on animal body weight were determined as follows. Male NOD-SCID mice bearing subcutaneous VCaP tumors were castrated when tumor volume was ~100mm³. Seven days after castration, the hosts were injected intravenously with 50mg/kg body weight of comparative compound (Ac), compound 1, compound 1c or compound 1d every other day for a total of seven doses. The experiment ended two days after the last dose. Change in body weight represents the change at day 14 compared to the day of 1^{st} injection of compounds. DMSO was used as the vehicle control. Results are presented in Figure 5.

### EXAMPLE 9

### ACTIVITY OF COMPARATIVE COMPOUNDS

For purposes of comparison, the activity of a compound lacking the chloro and flouro substitution of Formula I, and each of its stereoisomers, was determiend. The comparative compounds had the following structures (A), (Aa), (Ab), (Ac) and (Ad):

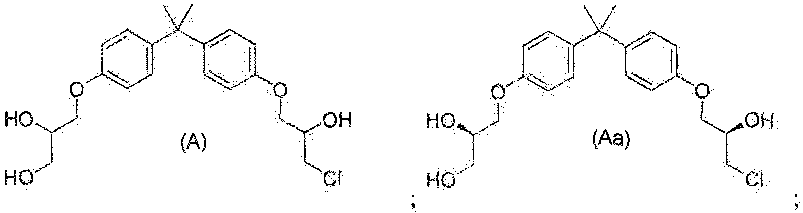

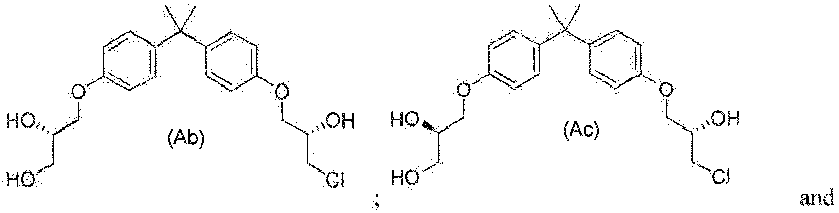

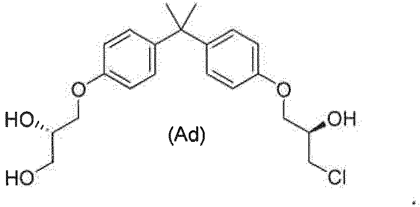

Dose response curves using PSA-luciferase reporter assay were used to calculate IC₅₀ values for comparative compound (A) and each of its stereoisomers. Reporter-specificity was investigated using AR-driven reporter gene constructs that included PSA-, probasin (PB) - and ARR3-luciferase reporters. All stereoisomers inhibited the transcriptional activity of AR as measured using these reporters. As seen in Table 3, comparative compound (Ac) has the best activity of all the comparative compounds.

**Table 3**

| Activity of Comparative Compounds | | | | |
|---|---|---|---|---|
| Compound | IC₅₀ (uM) mean±SD | PSA-Luc activity (%) mean±SD | PB-Luc activity (%) mean±SD | ARR3-Luc activity (%) mean±SD |
| (A) | 12.63±4.33 | 36.91±16.46 | 63.96±13.70 | 63.96±14.57 |
| (Aa) | 13.78±0.66 | 45.16±29.13 | 66.44±12.47 | 66.44±31.59 |
| (Ab) | 10.95±4.79 | 24.49±13.20 | 43.70±9.98 | 43.68±15.16 |
| (Ac) | 7.40±1.46* | 24.33±13.63 | 41.58±6.63 | 41.58±14.82 |
| (Ad) | 17.08±3.49 | 33.55±11.31 | 54.72±7.83 | 54.73±12.99 |

| | | | | |
|---|---|---|---|---|
| *(Ac) vs (Ad) p=0.00571; (Ac) vs (Aa) p=0.00116 | | | | |

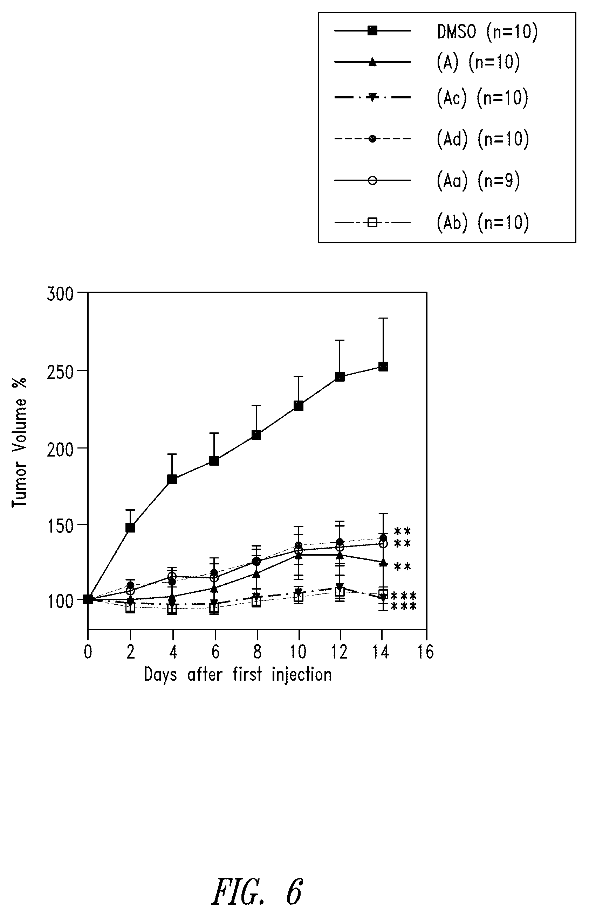

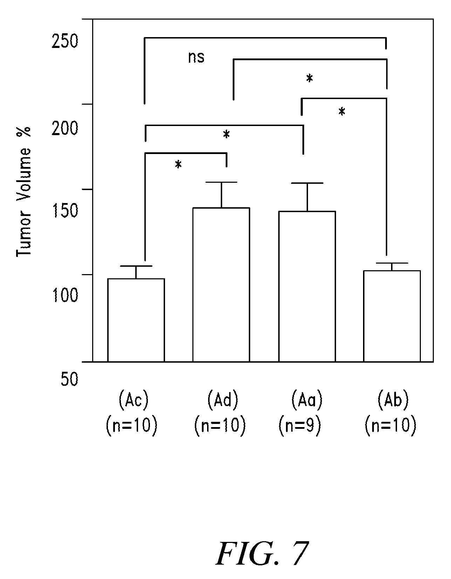

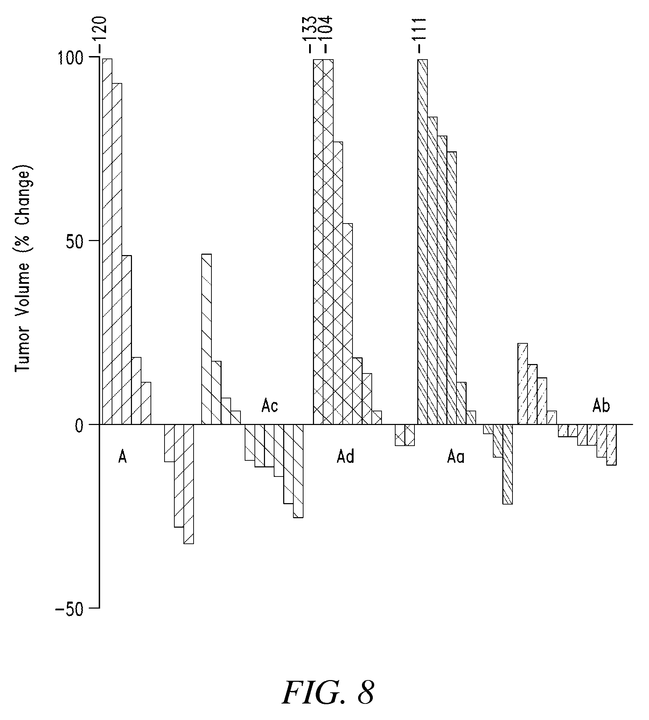

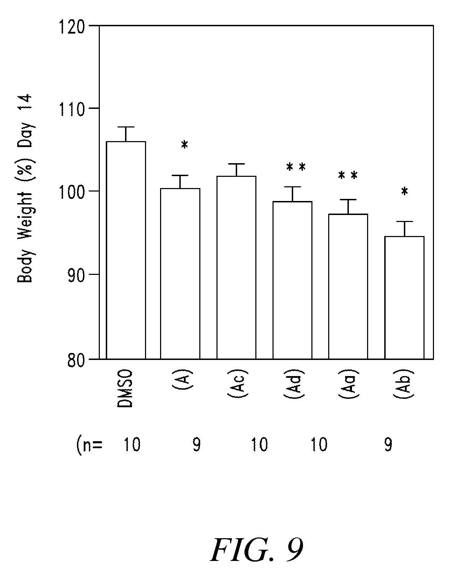

To determine if these *in vitro* responses could predict superior antitumor activity *in vivo,* the LNCaP CRPC xenograft model was employed. Data was obtained as described in Examples 5-8. All comparative compounds inhibited CRPC tumor growth compared to DMSO control (Fig. 6). Consistent with *in vitro* responses, comparative compound (Ac) as well as comparative compound (Ab) had better antitumor activity compared to comparative compounds (Ad) and (Aa) (Fig. 7). Tumor regression was attained in 60% of animals treated with (Ac) and (Ab), although (Ac) caused greater regression (Fig. 8). No loss of body weight was observed in animals treated with (Ac) while (Ab) had some losses (Fig. 9). Accordingly, comparative compound (Ac) is the most potent and least toxic of the comparative compounds

. Numeric ranges are inclusive of the numbers defining the range. The word "comprising" is used herein as an open-ended term, substantially equivalent to the phrase "including, but not limited to", and the word "comprises" has a corresponding meaning. As used herein, the singular forms "a", "an" and "the" include plural referents unless the context clearly dictates otherwise. Thus, for example, reference to "a thing" includes more than one such thing. Citation of references herein is not an admission that such references are prior art to the present invention.

## Claims

1. A compound having a structure of Formula I: 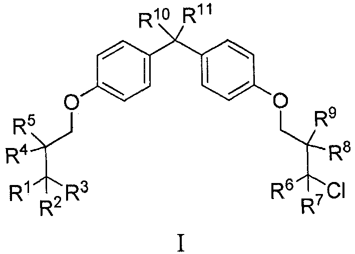 or a pharmaceutically acceptable salt or stereoisomer thereof, wherein:
R¹ is F, OH or OY;
R², R³ R⁵, R⁶, R⁷ and R⁹ are each independently H or F;
R⁴ is H, F, OH or OY;
R⁸ is OH or OY;
R¹⁰ and R¹¹ are each independently F or C₁-C₅ alkyl; and
Y is a moiety selected from: 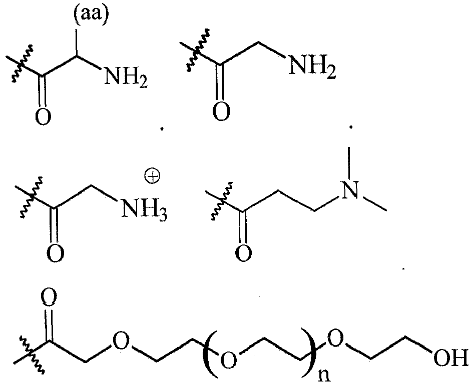 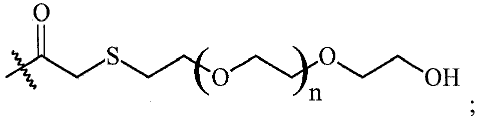 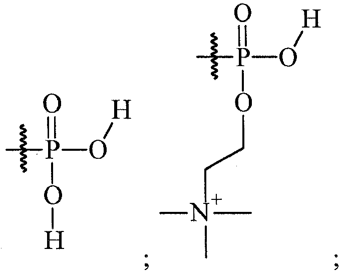 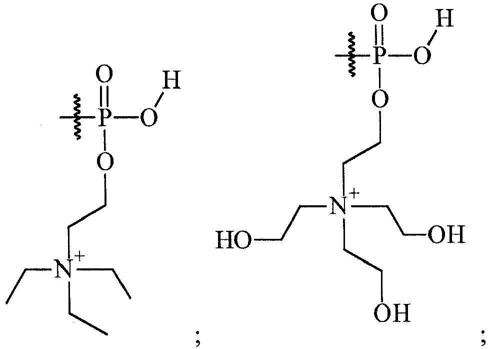 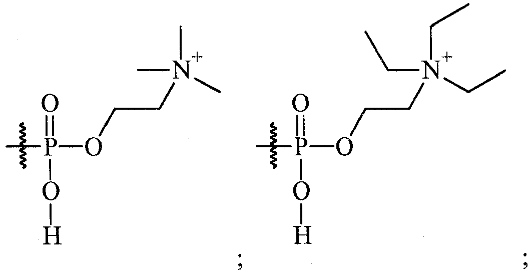 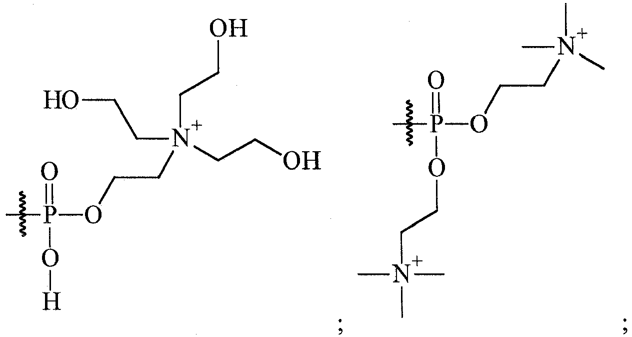 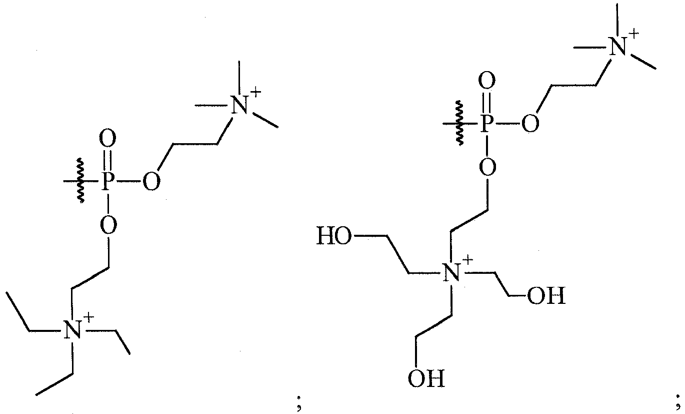 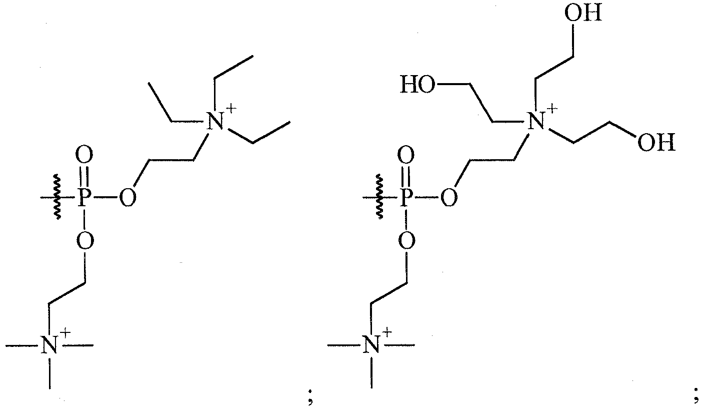 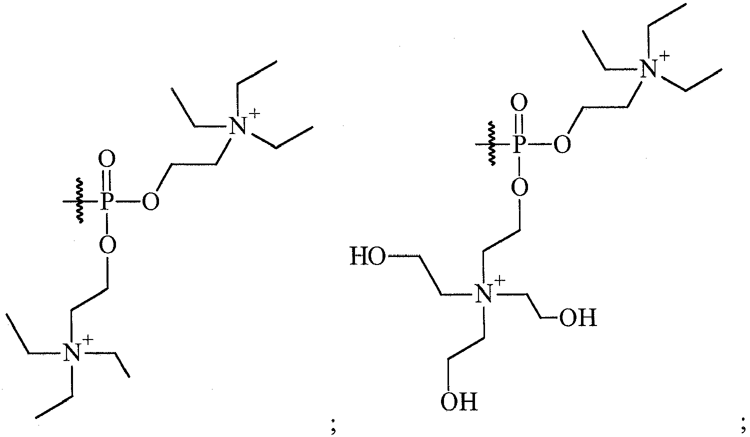 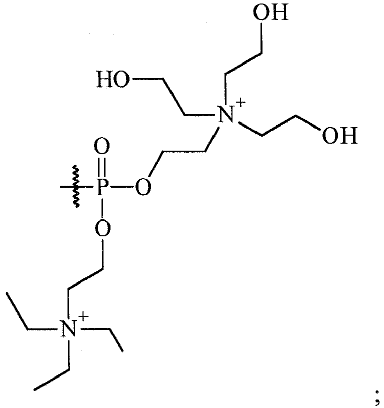 or 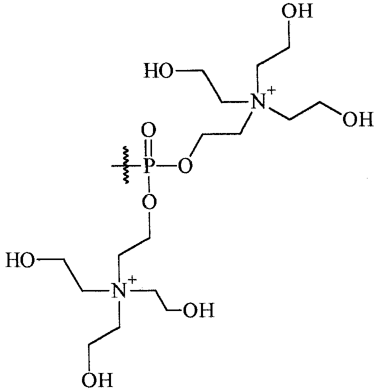
wherein (aa) is any naturally occurring amino acid side chain and
n is an integer between 1-200;
wherein at least one of R¹, R², R³, R⁴ or R⁵ is F.

2. The compound of claim 1, wherein the compound has one of the following structures (Ia), (Ib), (Ic) or (Id):

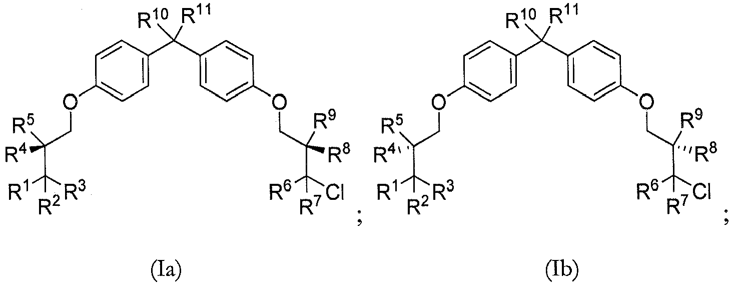

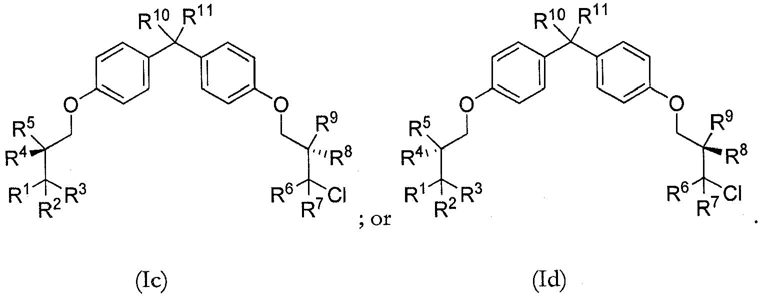

3. The compound of claim 1, wherein the compound has one of the following structures: 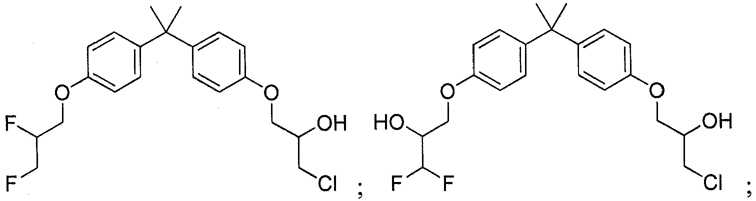 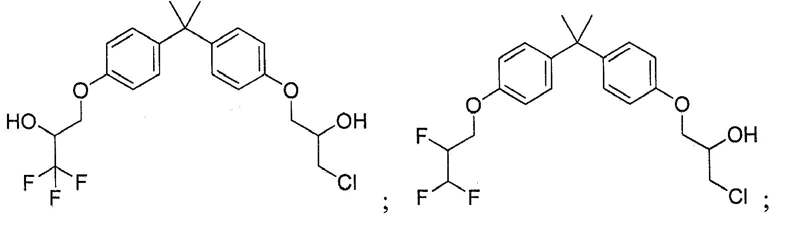 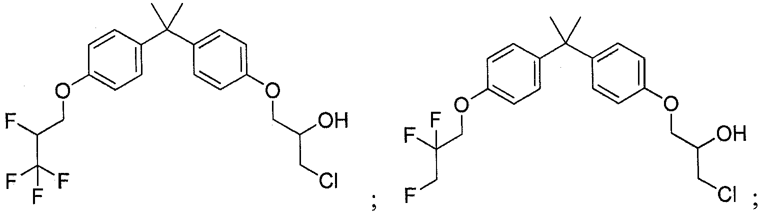 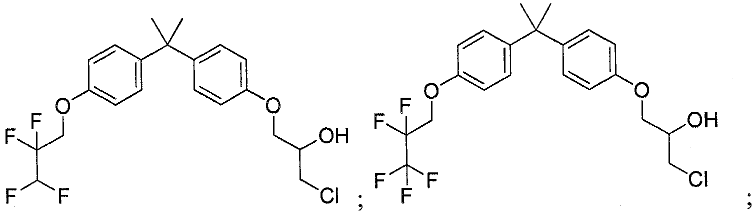 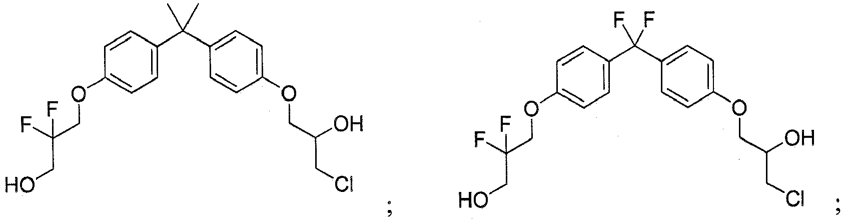 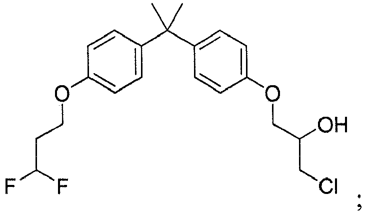 or 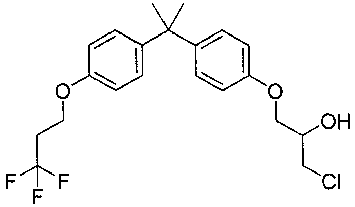 or a pharmaceutically acceptable salt or stereoisomer thereof.

4. The compound of claim 1, wherein the compound has the following structure (4): 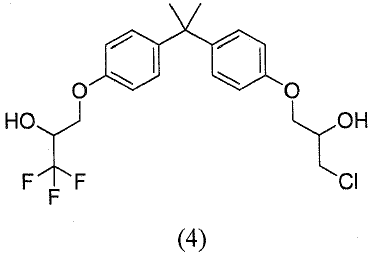 or a pharmaceutically acceptable salt or stereoisomer thereof.

5. The compound of claim 1, wherein the compound has the following structure (4a): 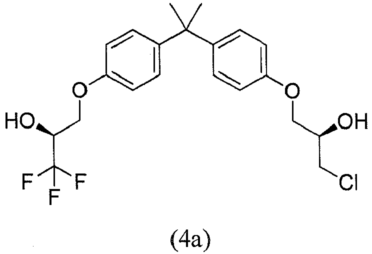 or a pharmaceutically acceptable salt thereof.

6. The compound of claim 1, wherein the compound has the following structure (4b): 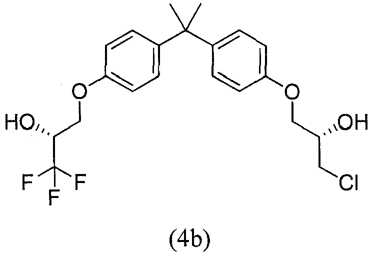 or a pharmaceutically acceptable salt thereof.

7. The compound of claim 1, wherein the compound has the following structure (4c): 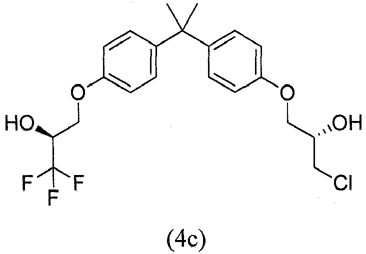 or a pharmaceutically acceptable salt thereof.

8. The compound of claim 1, wherein the compound has the following structure (4d): 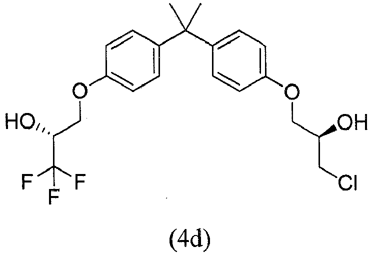 or a pharmaceutically acceptable salt thereof.

9. A compound, or pharmaceutically acceptable salt thereof, of any one of claims 1 to 8 for use in modulating androgen receptor (AR) activity for assays and research purposes.

10. A compound, or pharmaceutically acceptable salt thereof, of any one of claims 1 to 8 for use in modulating androgen receptor (AR) activity, optionally wherein modulating androgen receptor (AR) activity is for the treatment of one or more of the following: prostate cancer, breast cancer, ovarian cancer, endometrial cancer, salivary gland carcinoma, hair loss, acne, hirsutism, ovarian cysts, polycystic ovary disease, precocious puberty, spinal and bulbar muscular atrophy, and age-related macular degeneration.

11. The compound for the use of claim 10, wherein the indication is prostate cancer.

12. A pharmaceutical composition comprising a compound of any one of claims 1 to 8 and a pharmaceutically acceptable carrier.

13. A pharmaceutical composition comprising a compound of any one of claims 1 to 8, an additional therapeutic agent and a pharmaceutically acceptable carrier,-wherein the additional therapeutic agent is for treating prostate cancer, breast cancer, ovarian cancer, endometrial cancer, salivary gland carcinoma, hair loss, acne, hirsutism, ovarian cysts, polycystic ovary disease, precocious puberty, spinal and bulbar muscular atrophy or age-related macular degeneration, and wherein the additional therapeutic agent is MDV3100 , TAK 700, TOK 001; ARN-509; abiraterone acetate, bicalutamide, nilutamide, flutamide, cyproterone acetate, docetaxel, Bevacizumab (Avastin), OSU-HDAC42, VITAXIN, sunitumib, ZD-4054, Cabazitaxel (XRP-6258), MDX-010 (Ipilimumab), OGX 427, OGX Oil, finasteride, dutasteride, turosteride, bexlosteride, izonsteride, FCE 28260, SKF105,111 or a related compound thereof.

14. A pharmaceutical composition of any one of claims 12 to 13 for use in modulating androgen receptor (AR) activity.

15. The composition for the use of claim 14, wherein modulating androgen receptor (AR) activity is for the treatment of one or more of the following: prostate cancer, breast cancer, ovarian cancer, endometrial cancer, salivary gland carcinoma, hair loss, acne, hirsutism, ovarian cysts, polycystic ovary disease, precocious puberty, spinal and bulbar muscular atrophy, and age-related macular degeneration.

## Patentansprüche

1. Eine Verbinding mit einer Struktur von Formel I: 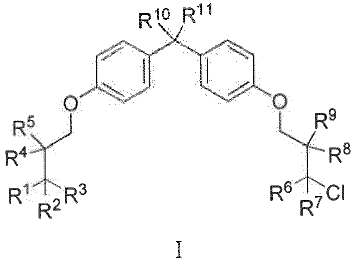 oder ein pharmazeutisch annehmbares Salz oder Stereoisomer davon, wobei:
R¹ F, OH oder OY ist;
R², R³ R⁵, R⁶, R⁷ und R⁹ jeweils unabhängig H oder F sind;
R⁴ H, F, OH oder OY ist;
R⁸ OH oder OY ist;
R¹⁰ und R¹¹ jeweils unabhängig F oder C₁-C₅-Alkyl sind; und
Y ein Rest ausgewählt aus: 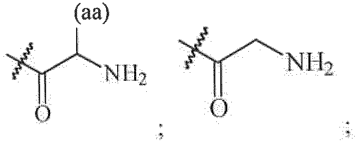 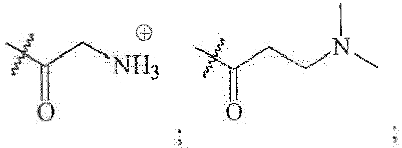 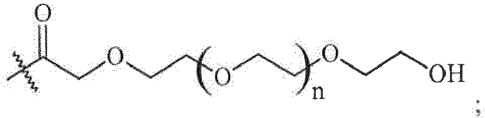 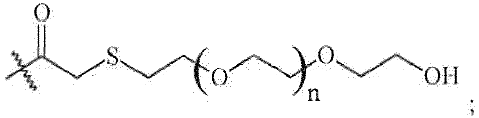 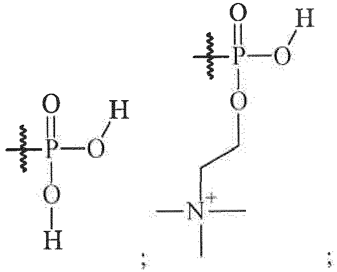 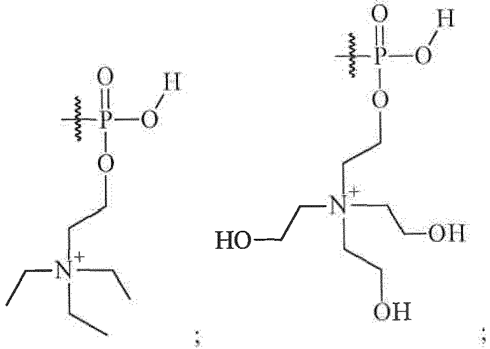 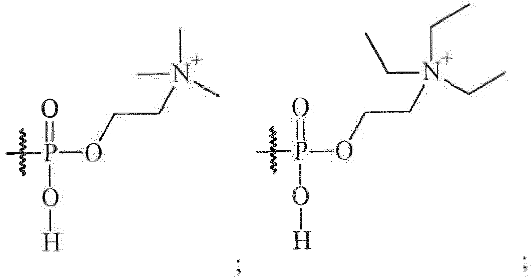 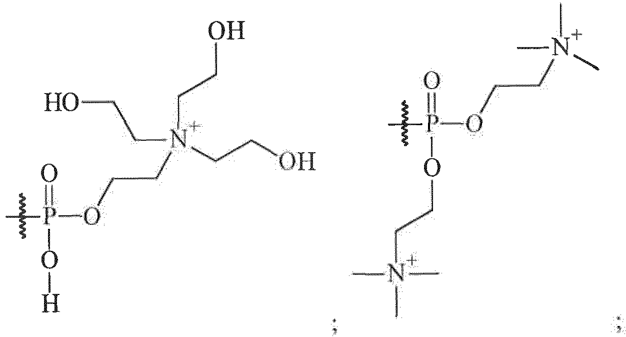 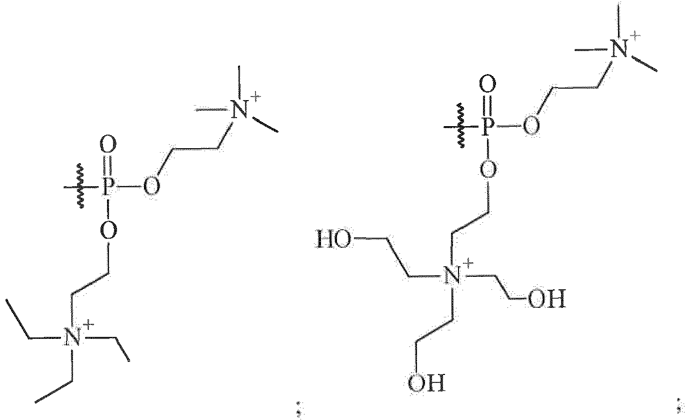 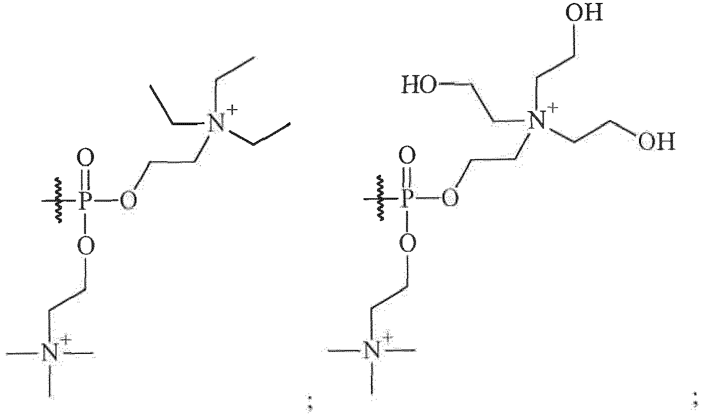 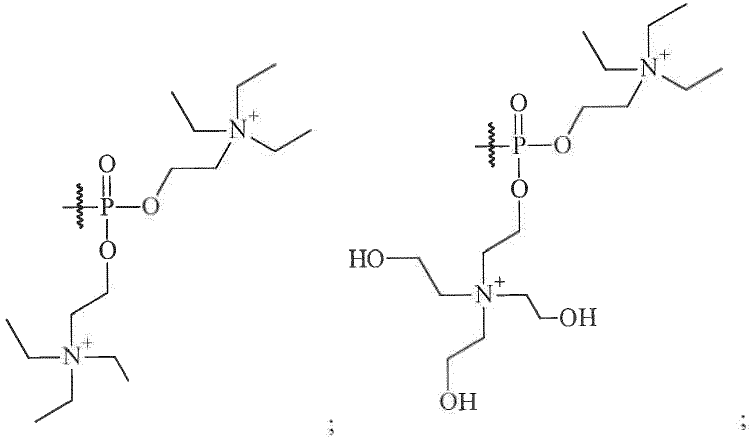 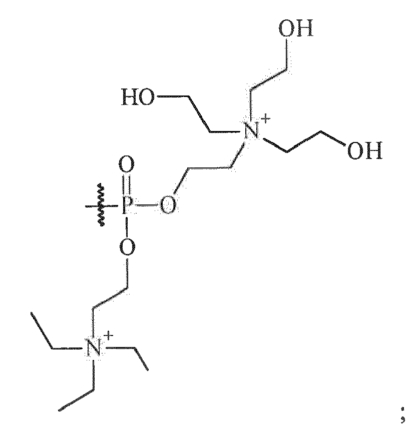 oder 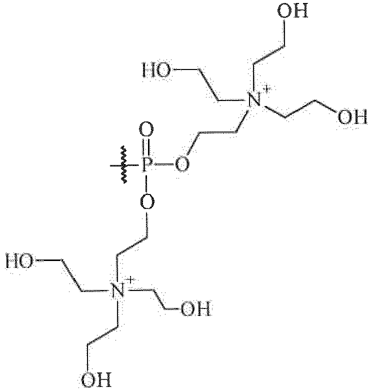 ist,
wobei (aa) jegliche natürlich vorkommende Aminosäure-Seitenkette ist und
n eine ganze Zahl zwischen 1-200 ist;
wobei mindestens eins von R¹, R², R³, R⁴ oder R⁵ F ist.

2. Die Verbindung von Anspruch 1, wobei die Verbindung eine der folgenden Strukturen (Ia), (Ib), (Ic) oder (Id) hat:

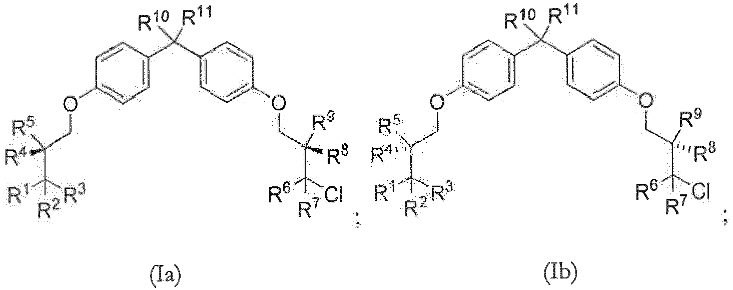

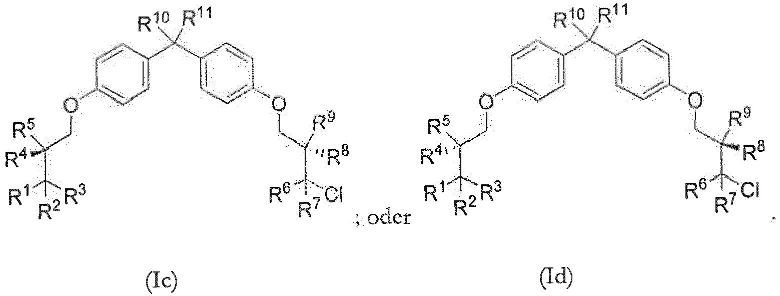

3. Die Verbindung von Anspruch 1, wobei die Verbindung eine der folgenden Strukturen hat: 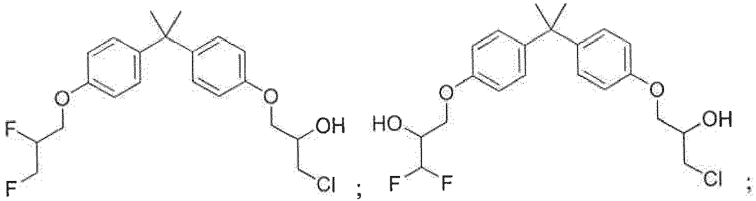 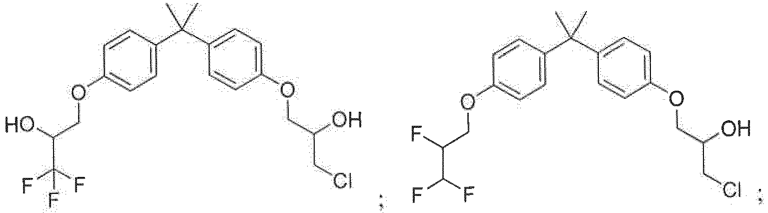 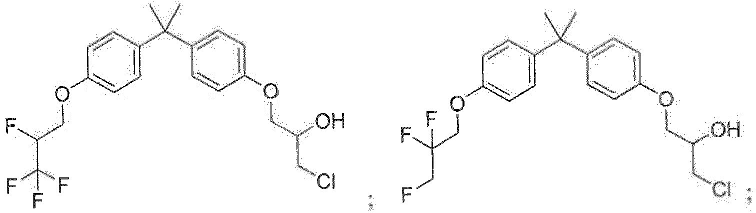 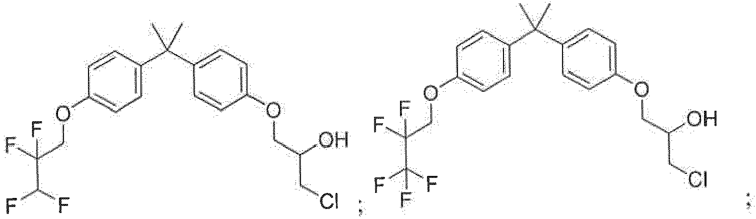 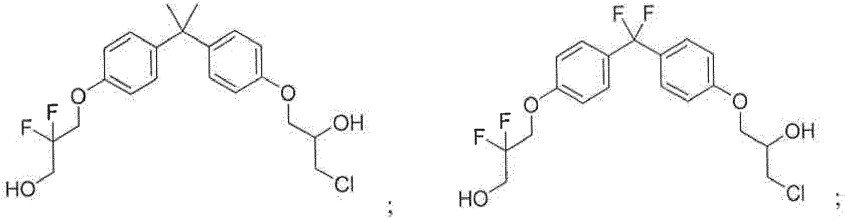 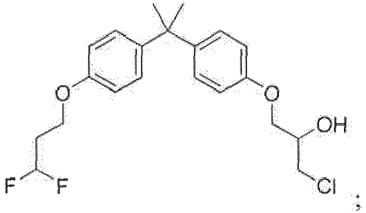 oder 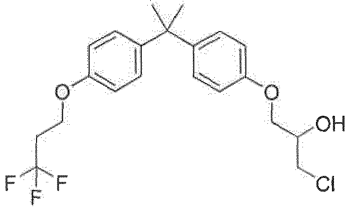 oder ein pharmazeutisch annehmbares Salz oder Stereoisomer davon.

4. Die Verbindung von Anspruch 1, wobei die Verbindung die folgende Struktur (4) hat: 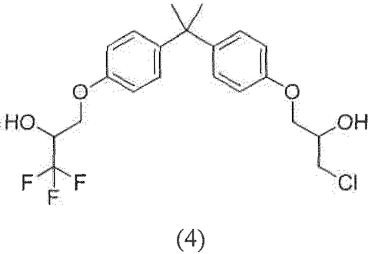 oder ein pharmazeutisch annehmbares Salz oder Stereoisomer davon.

5. Die Verbindung von Anspruch 1, wobei die Verbindung die folgende Struktur (4a) hat: 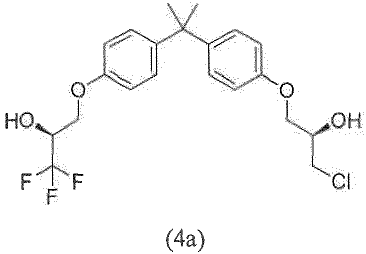 oder ein pharmazeutisch annehmbares Salz davon.

6. Die Verbindung von Anspruch 1, wobei die Verbindung die folgende Struktur (4b) hat: 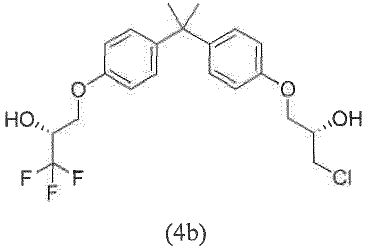 oder ein pharmazeutisch annehmbares Salz davon.

7. Die Verbindung von Anspruch 1, wobei die Verbindung die folgende Struktur (4c) hat: 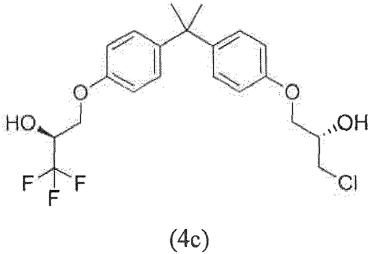 oder ein pharmazeutisch annehmbares Salz davon.

8. Die Verbindung von Anspruch 1, wobei die Verbindung die folgende Struktur (4d) hat: 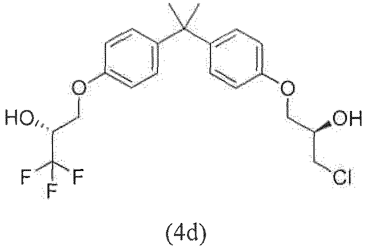 oder ein pharmazeutisch annehmbares Salz davon.

9. Eine Verbindung, oder ein pharmazeutisch annehmbares Salz davon, von einem der Ansprüche 1 bis 8 zur Verwendung bei der Modulation der Androgenrezeptor (AR)-Aktivität für Analysen und Forschungszwecke.

10. Eine Verbindung, oder ein pharmazeutisch annehmbares Salz davon, von einem der Ansprüche 1 bis 8 zur Verwendung bei der Modulation der Androgenrezeptor (AR)-Aktivität, wobei die Modulation der Androgenrezeptor (AR)-Aktivität optional zur Behandlung von einem oder mehreren der Folgenden dient: Prostata-Krebs, Brust-Krebs, Eierstock-Krebs, Gebärmutterschleimhaut-Krebs, Speicheldrüsen-Karzinom, Haarausfall, Akne, Hirsutismus, Eierstock-Zysten, polyzystischer Eierstockerkrankung, vorzeitiger Pubertät, spinaler und bulbärer Muskelatrophie und altersbedingter Makuladegeneration.

11. Die Verbindung zur Verwendung von Anspruch 10, wobei die Indikation Prostata-Krebs ist.

12. Eine pharmazeutische Zusammensetzung, die eine Verbindung von einem der Ansprüche 1 bis 8 und einen pharmazeutisch annehmbaren Träger umfasst.

13. Eine pharmazeutische Zusammensetzung, die eine Verbindung von einem der Ansprüche 1 bis 8, einen zusätzlichen therapeutischen Wirkstoff und einen pharmazeutisch annehmbaren Träger umfasst, wobei der zusätzliche therapeutische Wirkstoff der Behandlung von Prostata-Krebs, Brust-Krebs, Eierstock-Krebs, Gebärmutterschleimhaut-Krebs, Speicheldrüsen-Karzinom, Haarausfall, Akne, Hirsutismus, Eierstock-Zysten, polyzystischer Eierstockerkrankung, vorzeitiger Pubertät, spinaler und bulbärer Muskelatrophie oder altersbedingter Makuladegeneration dient und wobei der zusätzliche therapeutische Wirkstoff MDV3100 , TAK 700, TOK 001; ARN-509; Abirateron-Acetat, Bicalutamid, Nilutamid, Flutamid, Cyproteron-Acetat, Docetaxel, Bevacizumab (Avastin), OSU-HDAC42, VITAXIN, Sunitumib, ZD-4054, Cabazitaxel (XRP-6258), MDX-010 (Ipilimumab), OGX 427, OGX O1 l, Finasterid, Dutasterid, Turosterid, Bexlosterid, Izonsterid, FCE 28260, SKF105,111 oder eine damit verwandte Verbindung ist.

14. Eine pharmazeutische Zusammensetzung von einem der Ansprüche 12 bis 13 zur Verwendung bei der Modulation der Androgenrezeptor (AR)-Aktivität.

15. Die Zusammensetzung zur Verwendung von Anspruch 14, wobei die Modulation der Androgenrezeptor (AR)-Aktivität zur Behandlung von einem oder mehreren der Folgenden dient: Prostata-Krebs, Brust-Krebs, Eierstock-Krebs, Gebärmutterschleimhaut-Krebs, Speicheldrüsen-Karzinom, Haarausfall, Akne, Hirsutismus, Eierstock-Zysten, polyzystischer Eierstockerkrankung, vorzeitiger Pubertät, spinaler und bulbärer Muskelatrophie und altersbedingter Makuladegeneration.

## Revendications

1. Composé ayant une structure de formule I : 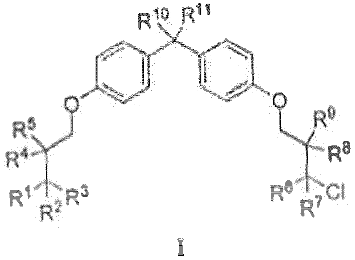 ou un sel ou stéréoisomère pharmaceutiquement acceptable de celui-ci, dans lequel :
R¹ est F, OH ou OY ;
R², R³, R⁵, R⁶, R⁷, R⁹ sont chacun indépendamment H ou F ;
R⁴ est H, F, OH ou OY ;
R⁸ e s t OH ou OY ;
R¹⁰ et R¹¹ sont chacun indépendamment F ou alkyle en C₁-C₅ ; et
Y est un fragment choisi parmi : 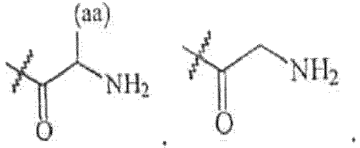 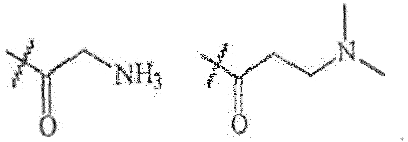 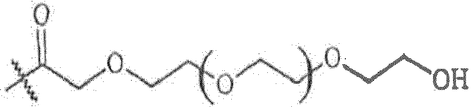 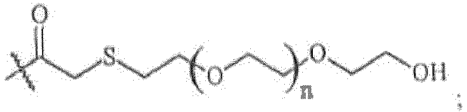 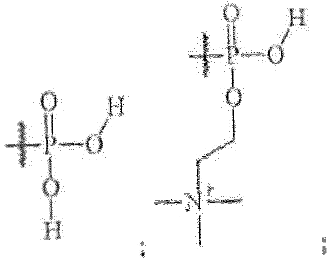 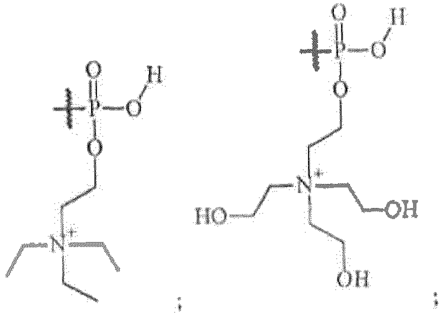 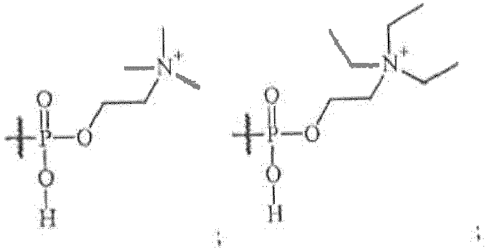 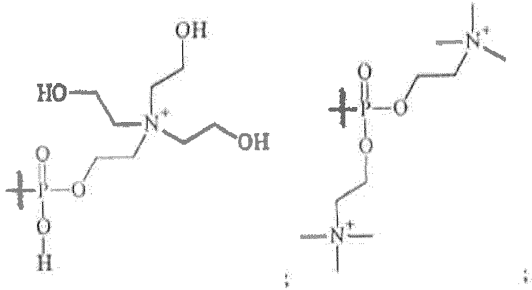 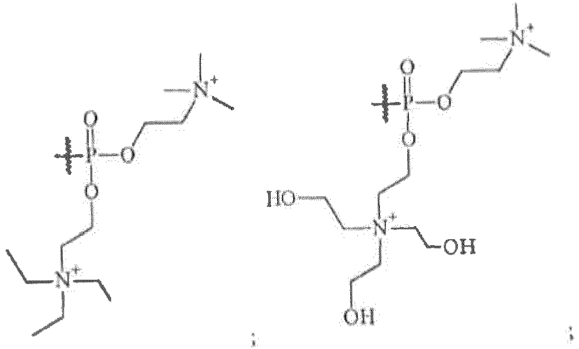 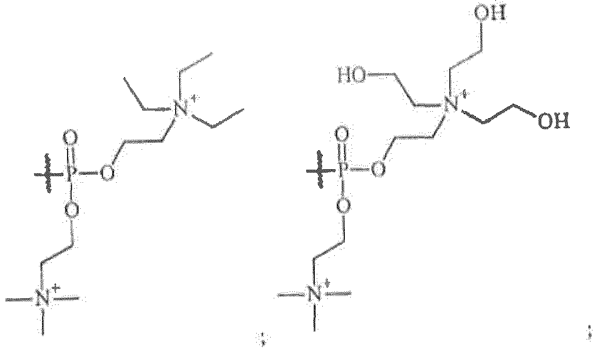 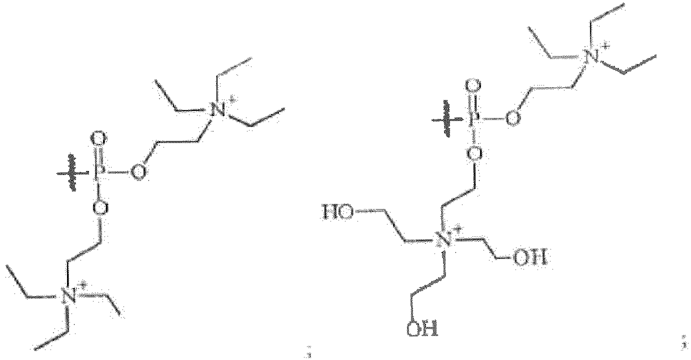 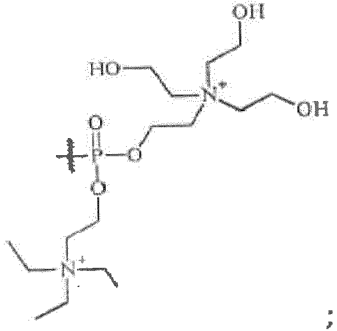 ou 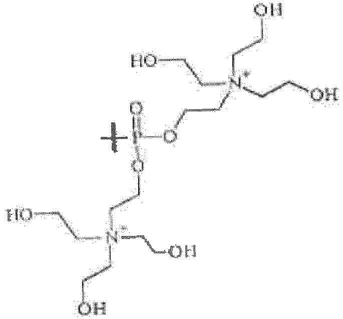
dans lequel (aa) est n'importe quelle chaîne latérale d'acide aminé d'origine naturelle et n est un entier entre 1 et 200 ;
dans lequel au moins un de R¹, R², R³, R⁴ ou R⁵ est F.

2. Composé selon la revendication 1, le composé ayant l'une des structures (Ia), (Ib), (Ic) ou (Id) suivantes :

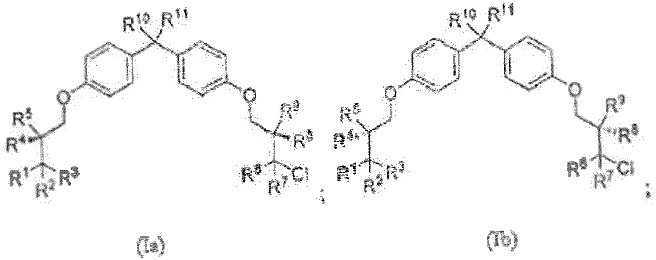

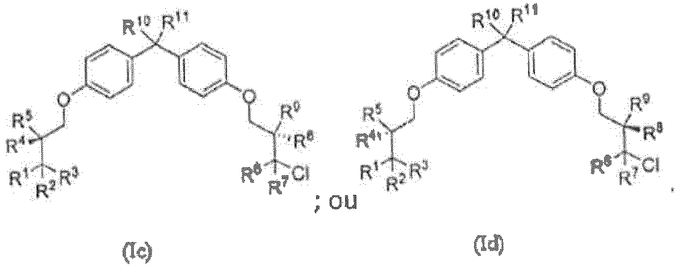

3. Composé selon la revendication 1, le composé ayant l'une des structures suivantes : 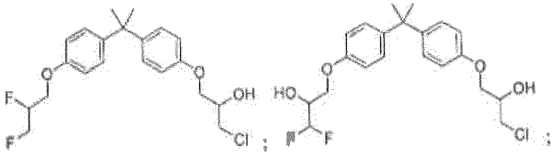 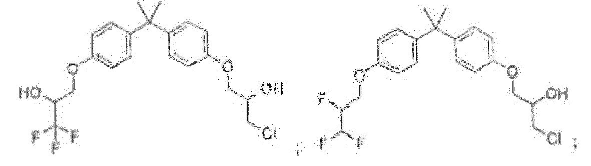 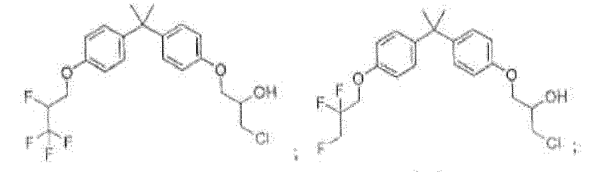 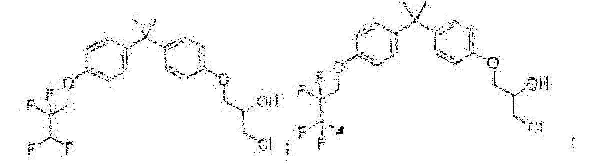 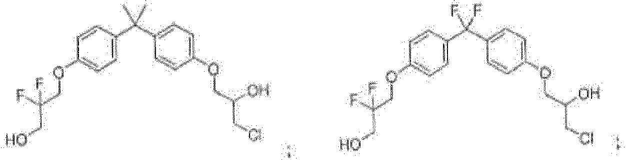 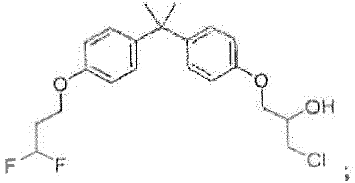 ou 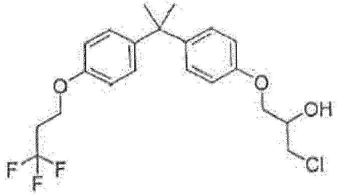 ou un sel ou stéréoisomère pharmaceutiquement acceptable de celui-ci.

4. Composé selon la revendication 1, le composé ayant la structure (4) suivante : 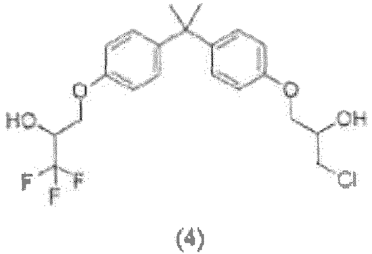 ou un sel ou stéréoisomère pharmaceutiquement acceptable de celui-ci.

5. Composé selon la revendication 1, le composé ayant la structure (4a) suivante : 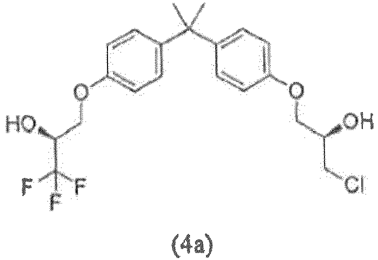 ou un sel pharmaceutiquement acceptable de celui-ci.

6. Composé selon la revendication 1, le composé ayant la structure (4b) suivante : 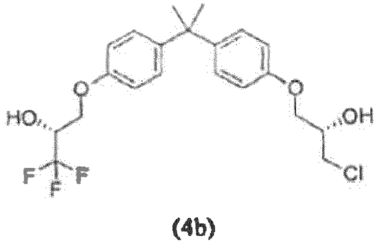 ou un sel pharmaceutiquement acceptable de celui-ci.

7. Composé selon la revendication 1, le composé ayant la structure (4c) suivante : 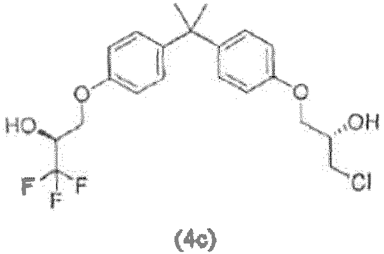 ou un sel pharmaceutiquement acceptable de celui-ci.

8. Composé selon la revendication 1, le composé ayant la structure (4d) suivante : 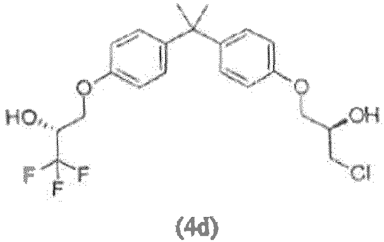 ou un sel pharmaceutiquement acceptable de celui-ci.

9. Composé, ou sel pharmaceutiquement acceptable de celui-ci, selon l'une quelconque des revendications 1 à 8 pour une utilisation dans la modulation de l'activité du récepteur d'androgène (RA) à des fins d'essais et de recherches.

10. Composé, ou sel pharmaceutiquement acceptable de celui-ci, selon l'une quelconque des revendications 1 à 8 pour une utilisation dans la modulation de l'activité du récepteur d'androgène (RA), éventuellement dans lequel la modulation de l'activité du récepteur d'androgène (RA) est destinée au traitement d'un ou plusieurs des états suivants : le cancer de la prostate, le cancer du sein, le cancer de l'ovaire, le cancer de l'endomètre, le carcinome des glandes salivaires, la perte de cheveux, l'acné, l'hirsutisme, les kystes ovariens, la maladie des ovaires polykystiques, la puberté précoce, l'atrophie musculaire spinale et bulbaire et la dégénérescence maculaire liée à l'âge.

11. Composé destiné à une utilisation selon la revendication 10, dans lequel l'indication est le cancer de la prostate.

12. Composition pharmaceutique comprenant un composé selon l'une quelconque des revendications 1 à 8 et un support pharmaceutiquement acceptable.

13. Composition pharmaceutique comprenant un composé selon l'une quelconque des revendications 1 à 8, un agent thérapeutique complémentaire et un support pharmaceutiquement acceptable, dans laquelle l'agent thérapeutique complémentaire est destiné à traiter le cancer de la prostate, le cancer du sein, le cancer de l'ovaire, le cancer de l'endomètre, le carcinome des glandes salivaires, la perte de cheveux, l'acné, l'hirsutisme, les kystes ovariens, la maladie des ovaires polykystiques, la puberté précoce, l'atrophie musculaire spinale et bulbaire et la dégénérescence maculaire liée à l'âge, et dans laquelle l'agent thérapeutique complémentaire est MDV3100, TAK 700, TOK 001 ; ARN-509 ; acétate d'abiratérone, bicalutamide, nilutamide, flutamide, acétate de cyproténone, docétaxel, Bevacizumab (Avastin), OSU-HDAC42, VITAXIN, sunitumib, ZD-4054, Cabazitaxel (XRP-6258), MDX-010 (Ipilimumab), OGX 427, OGX 011, finastéride, dutastéride, turostéride, bexlostéride, izonstéride, FCE 28260, SKF105,111 ou un composé associé à celui-ci.

14. Composition pharmaceutique selon l'une quelconque des revendications 12 et 13 pour une utilisation dans la modulation de l'activité du récepteur d'androgène (RA).

15. Composition pour une utilisation selon la revendication 14, dans laquelle la modulation de l'activité du récepteur d'androgène (RA) est destinée au traitement d'un ou plusieurs des états suivante : le cancer de la prostate, le cancer du sein, le cancer de l'ovaire, le cancer de l'endomètre, le carcinome des glandes salivaires, la perte de cheveux, l'acné, l'hirsutisme, les kystes ovariens, la maladie des ovaires polykystiques, la puberté précoce, l'atrophie musculaire spinale et bulbaire et la dégénérescence maculaire liée à l'âge.
